(19) **Europäisches Patentamt / European Patent Office / Office européen des brevets**

(11) **EP 1 384 403 B1**

(12) **EUROPEAN PATENT SPECIFICATION**

(45) Date of publication and mention of the grant of the patent:
**03.01.2007 Bulletin 2007/01**

(51) Int Cl.:
*A01N 37/18* (2006.01)   *A61F 13/00* (2006.01)
*A61K 39/00* (2006.01)   *A61K 9/127* (2006.01)
*A61K 9/52* (2006.01)   *A61K 9/56* (2006.01)

(21) Application number: **03017154.0**

(22) Date of filing: **14.11.1997**

(54) **Adjuvant for transcutaneous immunization**

Adjuvant für transkutane Immunisation

Adjuvant pour immunisation transcutanée

(84) Designated Contracting States:
**AT BE CH DE DK ES FI FR GB GR IE IT LI LU MC NL PT SE**

(30) Priority: **14.11.1996 US 749164**
**17.07.1997 US 896085**

(43) Date of publication of application:
**28.01.2004 Bulletin 2004/05**

(60) Divisional application:
**05002995.8 / 1 557 177**

(62) Document number(s) of the earlier application(s) in accordance with Art. 76 EPC:
**97947608.2 / 1 014 787**

(73) Proprietor: **The Government of the United States of America, as represented by The Secretary of the Army Frederick, MD 21701-5012 (US)**

(72) Inventors:
• **Glenn, Gregory**
**Giatherburg,MD 20878 (US)**

• **Alving, Carl**
**Bethesda, MD 20817 (US)**

(74) Representative: **Lipscombe, Martin John et al Keith W Nash & Co, Pearl Assurance House, 90-92 Regent Street Cambridge CB2 1DP (GB)**

(56) References cited:
**WO-A-88/06626**   **WO-A-95/17211**

• **K. KRUEGER ET AL.: "The family of bacterial ADP-ribosylating exotoxins." CLINICAL MICROBIOLOGY REVIEWS, vol. 8, no. 1, January 1995 (1995-01), pages 34-47, XP001146599 USA**

Remarks:
The file contains technical information submitted after the application was filed and not included in this specification

**Description**

BACKGROUND OF THE INVENTION

The invention relates to transcutaneous immunization, and adjuvants useful therein, to induce an antigen-specific immune response.

Transcutaneous immunization requires both passage of an antigen through the outer barriers of the skin, which are normally impervious to such passage, and an immune response to the antigen. In U.S. Appln. No. 08/749,164, use of cholera toxin as an antigen was shown to elicit a strong antibody response that is highly reproducible; the antigen could be applied in a saline solution to the skin, with or without liposomes. In the present application, we show transcutaneous immunization using adjuvants such as, for example, bacterial exotoxins, their subunits, and related toxins.

There is a report of transdermal immunization with transferosomes by Paul et al. (1995). In this publication, the transferosomes are used as a carrier for proteins (bovine serum albumin and gap junction proteins) against which the complement-mediated lysis of antigen-sensitized liposomes is directed. An immune response was not induced when solution containing the protein was placed on the skin; only transferosomes were able to transport antigen across the skin and achieve immunization. As discussed in U.S. Appln. No. 08/749,164, transferosomes are not liposomes.

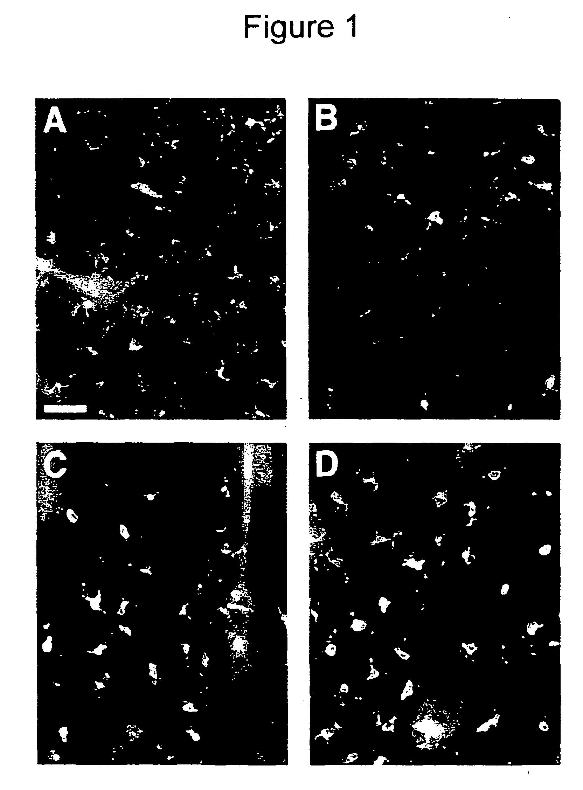

Figure 1 of Paul et al. (1995) showed that only a formulation of antigen and transferosomes induced an immune response, assayed by lysis of antigen-sensitized liposomes. Formulations of antigen in solution, antigen and mixed micelles, and antigen and liposomes (i.e., smectic mesophases) applied to the skin did not induce an immune response equivalent to that induced by subcutaneous injection. Therefore, there was a positive control (i.e., antigen and transfersomes) to validate their negative conclusion that a formulation of antigen and liposomes did not cause transdermal immunization.

Paul et al. (1995) stated on page 3521 that the skin is an effective protective barrier that is "impenetrable to substances with a molecular mass at most 750 DA", precluding non-invasive immunization with large immunogen through intact skin. Therefore, the reference would teach away from using a molecule like cholera toxin (which is 85,000 daltons) because such molecules would not be expected to penetrate the skin and, therefore, would not be expected to achieve immunization. Thus, skin represents a barrier that would make penetration by an adjuvant or antigen like cholera toxin unexpected without the disclosure of the present invention.

Paul and Cevc (1995) stated on page 145, "Large molecules normally do not get across the intact mammalian skin. It is thus impossible to immunize epicutaneously with simple peptide or protein solutions." They concluded, "The dermally applied liposomal or mixed micellar immunogens are biologically as inactive as simple protein solutions, whether or not they are combined with the immunoadjuvant lipid A."

Wang et al. (1996) placed a solution of ovalbumin (OVA) in water on the skin of shaved mice to induce an allergic type response as a model for atopic dermatitis. Mice were anesthetized and covered with an occlusive patch containing up to 10 mg of OVA, which was placed on the skin continuously for four days. This procedure was repeated after two weeks.

In Figure 2 of Wang et al. (1996), an ELISA assay done to determine the IgG2a antibody response showed no IgG2a antibody response to OVA. However, IgE antibodies that are associated with allergic responses could be detected. In a further experiment, the mice were more extensively patched with OVA in solution for four days every two weeks. This was repeated five times, i.e., the mice wore patches for a total of 20 days. Again, the high dose of OVA did not produce significant IgG2a antibodies. Significant levels of IgE antibodies were produced.

The authors stated on page 4079 that "we established an animal model to show that epicutaneous exposure to protein Ag, in the absence of adjuvant, can sensitize animals and induce a dominant Th2-like response with high levels of IgE". Extensive epicutaneous exposure to high doses of protein antigen could not produce significant IgG antibodies but could induce IgE antibodies, the hallmark of an allergic type reaction. Thus, Wang et al. (1996) teaches that OVA exposure as described is a model for atopic dermatitis and not a mode of immunization. Therefore, following the teaching of the reference, one would have expected that transcutaneous immunization with antigen would induce high levels of IgE antibodies if it were to pass through the skin and induce an immune response. Instead, we have unexpectedly found that antigen placed on the skin in a saline solution with adjuvant induces high levels of IgG and some IgA, but not IgE.

In contrast to the cited references, the inventors have found that application to the skin of antigen and adjuvant provides a transcutaneous delivery system for antigen that can induce an antigen-specific immune response of IgG or IgA. The adjuvant is preferably an ADP-ribosylating exotoxin. Optionally, hydration, penetration enhancers, or occlusive dressings may be used in the transcutaneous delivery system.

SUMMARY OF THE INVENTION

An object of the invention is to provide a system for transcutaneous immunization that induces an immune response (e.g., humoral and/or cellular effectors) in an animal or human.

The system provides simple application to intact skin of an organism of a formulation comprised of antigen and adjuvant to induce a specific immune response against the antigen.

In particular, the adjuvant may activate antigen presenting cells of the immune system (e.g., Langerhans cells in the epidermis, dermal dendritic cells, dendritic cells, macrophages, B lymphocytes) and/or induce the antigen presenting cells to phagocytose the antigen. The antigen presenting cells then present the antigen to T and B cells. In the instance of Langerhans cells, the antigen presenting cells then may migrate from the skin to the lymph nodes and present antigen to lymphocytes (e.g., B and/or T cells), thereby inducing an antigen-specific immune response.

In addition to eliciting immune reactions leading to generation of an antigen-specific B lymphocyte and/or T lymphocyte, including a cytotoxic T lymphocyte (CTL), another object of the invention is to positively and/or negatively regulate components of the immune system by using the transcutaneous immunization system to affect antigen-specific helper T lymphocytes (Th1, Th2 or both)

A first embodiment of the invention concerns the an antigen and an adjuvant, in the manufacture of a formulation for Transcutaneous immunization of a subject wherein said formulation induces an immune response specific for the antigen following application of the formulation to the subject's skin, and further wherein the formulation does not include a penetration enhancer. The formulation may include additional antigens such that transcutaneous application of the formulation induces an immune response to multiple antigens. In such a case, the antigens may or may not be derived from the same source, but the antigens will have different chemical structures so as to induce immune responses specific for the different antigens. Antigen-specific lymphocytes may participate in the immune response and, in the case of participation by B lymphocytes, antigen-specific antibodies may be part of the immune response.

The above method is used to treat an organism. If the antigen is derived from a pathogen, the treatment vaccinates the organism against infection by the pathogen or against its pathogenic effects such as those caused by toxin secretion. A formulation that includes a tumor antigen may provide a cancer treatment, a formulation that includes an autoantigen may provide a treatment for a disease caused by the organism's own immune system (e.g., autoimmune disease), and a formulation that includes an allergen may be used in immunotherapy to treat an allergic disease.

In a second embodiment of the invention, a patch for use in the above methods is provided. The patch comprises a dressing, and effective amounts of antigen and adjuvant. The dressing may be occlusive or non-occlusive. The patch may include additional antigens such that application of the patch induces an immune response to multiple antigens. In such a case, the antigens may or may not be derived from the same source, but the antigens will have different chemical structures so as to induce an immune response specific for the different antigens. For effective treatment, multiple patches may be applied at frequent intervals or constantly over a period of time.

The formulation is applied to intact skin overlying more than one draining lymph node field using either single or multiple applications. The formulation may include additional antigens such that application to intact skin induces an immune response to multiple antigens. In such a case, the antigens may or may not be derived from the same source, but the antigens will have different chemical structures so as to induce an immune response specific for the different antigens.

The products and methods of the invention may be used to treat existing disease, to prevent disease, or to reduce the severity and/or duration of disease. However, induction of allergy, atopic disease, dermatitis, or contact hypersensitivity is not preferred.

In addition to antigen and adjuvant, the formulation may further comprise a hydrating agent (e.g., liposomes), a penetration enhancer, or both. For example, the antigen-adjuvant formulation may further comprise an emulsion made with AQUAPHOR (petrolatum, mineral oil, mineral wax, wool wax, panthenol, bisabol, and glycerin), emulsions (e.g., aqueous creams), oil-in-water emulsions (e.g., oily creams), anhydrous lipids and oil-in-water emulsions, anhydrous lipids and water-in-oil emulsions, fats, waxes, oil, silicones, humectants (e.g., glycerol), a jelly (e.g., SURGILUBE, KY jelly), or a combination thereof. The formulation may be provided as an aqueous solution. '

The formulation preferably does not include an organic solvent. The formulation may be applied after the skin has been swabbed with alcohol. However, removal of the keratinocyte layer prior to application of the formulation to the extent achieved with a depilatory agent is not preferred.

The antigen may be derived from a pathogen that can infect the organism (e.g., bacterium, virus, fungus, or parasite), or a cell (e.g., tumor cell or normal cell). The antigen may be a tumor antigen or an autoantigen. Chemically, the antigen may be a carbohydrate, glycolipid, glycoprotein, lipid, lipoprotein, phospholipid, polypeptide, or chemical or recombinant conjugate of the above. The molecular weight of the antigen may be greater than 500 daltons, preferably greater than 800 daltons, and more preferably greater than 1000 daltons.

Antigen may be obtained by recombinant means, chemical synthesis, or purification from a natural source. Preferred are proteinaceous antigen or conjugates with polysaccharide. Antigen may be at least partially purified in cell-free form. Alternatively, antigen may be provided in the form of a live virus, an attenuated live virus, or an inactivated virus.

Inclusion of an adjuvant may allow potentiation or modulation of the immune response. Moreover, selection of a suitable antigen or adjuvant may allow preferential induction of a humoral or cellular immune response, specific antibody isotypes (e.g., IgM, IgD, IgA1, IgA2, IgE, IgG1, IgG2, IgG3, IgG4, or a combination thereof), and/or specific T-cell subsets (e.g., CTL, Th1, Th2, $T_{DTH}$, or a combination thereof).

Preferably, the adjuvant is an ADP-ribosylating exotoxin or a subunit thereof. Optionally, an activator of Langerhans cells may be used.

Optionally, antigen, adjuvant, or both may be provided in the formulation by means of a nucleic acid (e.g., DNA, RNA, cDNA, cRNA) encoding the antigen or adjuvant as appropriate. This technique is called genetic immunization.

The term "antigen" as used in the invention, is meant to describe a substance that induces a specific immune response when presented to immune cells of an organism. An antigen may comprise a single immunogenic epitope, or a multiplicity of immunogenic epitopes recognized by a B-cell receptor (i.e., antibody on the membrane of the B cell) or a T-cell receptor. A molecule may be both an antigen and an adjuvant (e.g., cholera toxin) and, thus, the formulation may contain only one component.

The term "adjuvant" as used in the invention, is meant to describe a substance added to the formulation to assist in inducing an immune response to the antigen. A substance may act as both adjuvant and antigen by inducing both immunostimulation and a specific antibody or T-cell response.

The term "effective amount" as used in the invention, is meant to describe that amount of antigen which induces an antigen-specific immune response. Such induction of an immune response may provide a treatment such as, for example, immunoprotection, desensitization, immunosuppression, modulation of autoimmune disease, potentiation of cancer immunosurveillance, or therapeutic vaccination against an established infectious disease.

The term "draining lymph node field" as used in the invention means an anatomic area over which the lymph collected is filtered through a set of defined set of lymph nodes (e.g., cervical, axillary, inguinal, epitrochelear, popliteal, those of the abdomen and thorax).

BRIEF DESCRIPTION OF THE DRAWINGS

Figure 1 shows cholera toxin (CT) induces enhanced major histocompatibility complex (MHC) class II expression on Langerhans cells (LC), changes in LC morphology, and loss of LCs (presumably through migration). BALB/c mice ($H-2^d$) were transcutaneously immunized with 250 $\mu$g of cholera CT or its B subunit (CTB) in saline solution on the ear. Previous experiments had established that mice were readily immunized when using the skin of the ear (7000 anti-CT ELISA Units after a single immunization). After 16 hours, epidermal sheets were prepared and stained for MHC class II molecules (scale bar is 50 $\mu$m) . Panels indicate (A) saline alone as a negative control, (B) transcutaneous immunization with CT in saline, (C) transcutaneous immunization with CTB in saline, and (D) intradermal injection with tumor necrosis factor-$\alpha$ (10 $\mu$g) as a positive control.

DETAILED DESCRIPTION OF THE INVENTION

A transcutaneous immunization system delivers agents to specialized cells (e.g., antigen presentation cell, lymphocyte) that produce an immune response (Bos, 1997). These agents as a class are called antigens. Antigen may be composed of chemicals such as, for example, carbohydrate, glycolipid, glycoprotein, lipid, lipoprotein, phospholipid, polypeptide, protein, conjugates thereof, or any other material known to induce an immune response. Antigen may be provided as a whole organism such as, for example, a bacterium or virion; antigen may be obtained from an extract or lysate, either from whole cells or membrane alone; or antigen may be chemically synthesized or produced by recombinant means, or by inactivation of a virus.

Processes for preparing a pharmaceutical formulation are well-known in the art, whereby the antigen and adjuvant is combined with a pharmaceutically acceptable carrier vehicle. Suitable vehicles and their preparation are described, for example, in Remington's Pharmaceutical Sciences by E.W. Martin. Such formulations will contain an effective amount of the antigen and adjuvant together with a suitable amount of vehicle in order to prepare pharmaceutically acceptable compositions suitable for administration to a human or animal. The formulation may be applied in the form of an cream, emulsion, gel, lotion, ointment, paste, solution, suspension, or other forms known in the art. In particular, formulations that enhance skin hydration, penetration, or both are preferred. There may also be incorporated other pharmaceutically acceptable additives including, for example, diluents, binders, stabilizers, preservatives, and colorings.

Increasing hydration of the stratum corneum will increase the rate of percutaneous absorbtion of a given solute (Roberts and Walker, 1993). As used in the present invention, "penetration enhancer" does not include substances such as, for example: water, physiological buffers, saline solutions, and alcohols which would not perforate the skin.

An object of the present invention is to provide a novel means for immunization through intact skin without the need for perforating the skin. The transcutaneous immunization system provides a method whereby antigens and adjuvant can be delivered to the immune system, especially specialized antigen presentation cells underlying the skin such as, for example, Langerhans cells.

Without being bound to any particular theory but only to provide an explanation for our observations, it is presumed that the transcutaneous immunization delivery system carries antigen to cells of the immune system where an immune response is induced. The antigen may pass through the normal protective outer layers of the skin (i.e., stratum corneum) and induce the immune response directly, or through an antigen presenting cell (e.g., macrophage, tissue macrophage, Langerhans cell, dendritic cell, dermal dendritic cell, B lymphocyte, or Kupffer cell) that presents processed antigen to a T lymphocyte. Optionally, the antigen may pass through the stratum corneum via a hair follicle or a skin organelle (e.g., sweat gland, oil gland).

Transcutaneous immunization with bacterial ADP-ribosylating exotoxins (bAREs) may target the epidermal Langerhans cell, known to be among the most efficient of the antigen presenting cells (APCs) (Udey, 1997). We have found that bAREs activate Langerhans cells when applied epicutaneously to the skin in saline solution. The Langerhans cells direct specific immune responses through phagocytosis of the antigens, and migration to the lymph nodes where they act as APCs to present the antigen to lymphocytes (Udey, 1997), and thereby induce a potent antibody response. Although the skin is generally considered a barrier to invading organisms, the imperfection of this barrier is attested to by the numerous Langerhans cells distributed throughout the epidermis that are designed to orchestrate the immune response against organisms invading via the skin (Udey, 1997).

According to Udey (1997):

"Langerhans cells are bone-marrow derived cells that are present in all mammalian stratified squamous epithelia. They comprise all of the accessory cell activity that is present in uninflamed epidermis, an in the current paradigm are essential for the initiation and propagation of immune responses directed against epicutaneously applied antigens. Langerhans cells are members of a family of potent accessory cells ('dendritic cells') that are widely distributed, but infrequently represented, in epithelia and solid organs as well as in lymphoid tissue ...

"It is now recognized that Langerhans cells (and presumably other dendritic cells) have a life cycle with at least two distinct stages. Langerhans cells that are located in epidermis constitute a regular network of antigen-trapping 'sentinel' cells. Epidermal Langerhans cells can ingest particulates, including microorganisms, and are efficient processors of complex antigens. However, they express only low levels of MHC class I and II antigens and costimulatory molecules (ICAM-1, B7-1 and B7-2) and are poor stimulators of unprimed T cells. After contact with antigen, some Langerhans cells become activated, exit the epidermis and migrate to T-cell-dependent regions of regional lymph nodes where they local as mature dendritic cells. In the course of exiting the epidermis and migrating to lymph nodes, antigen-bearing epidermal Langerhans cells (now the 'messengers') exhibit dramatic changes in morphology, surface phenotype and function. In contrast to epidermal Langerhans cells, lymphoid dendritic cells are essentially non-phagocytic and process protein antigens inefficiently, but express high levels of MHC class I and class II antigens and various costimulatory molecules and are the most potent stimulators of naive T cells that have been identified."

We envision that the potent antigen presenting capability of the epidermal Langerhans cells can be exploited for transcutaneously delivered vaccines. A transcutaneous immune response using the skin immune system would require delivery of vaccine antigen only to Langerhans cells in the stratum corneum (the outermost layer of the skin consisting of cornified cells and lipids) via passive diffusion and subsequent activation of the Langerhans cells to take up antigen, migrate to B-cell follicles and/or T-cell dependent regions, and present the antigen to B and/or T cells (Stingl et al., 1989). If antigens other that bAREs (for example BSA) were to be phagocytosed by the Langerhans cells, then these antigens could also be taken to the lymph node for presentation to T-cells and subsequently induce an immune response specific for that antigen (e.g., BSA). Thus, a feature of transcutaneous immunization is the activation of the Langerhans cell, presumably by a bacterial ADP-ribosylating exotoxin, ADP-ribosylating exotoxin binding subunits (e.g., cholera toxin B subunit), or other Langerhans cell activating substance.

The mechanism of transcutaneous immunization via Langerhans cells activation, migration and antigen presentation is clearly shown by the upregulation of MHC class II expression in the epidermal Langerhans cells from epidermal sheets transcutaneously immunized with CT or CTB. In addition, the magnitude of the antibody response induced by transcutaneous immunization and isotype switching to predominantly IgG is generally achieved with T-cell help stimulated by antigen presenting cells such as Langerhans cells or dendritic cells (Janeway and Travers, 1996), and activation of both Th1 and Th2 pathways as suggested by the production of IgG1 and IgG2a (Paul and Seder, 1994; Seder and Paul, 1994). Additionally, T cell proliferation to the antigen OVA is shown in mice immunized with CT + OVA. Alternatively, a large antibody response may be induced by a thymus-independent antigen type 1 (TI-1) which directly activates the B cell (Janeway and Travers, 1996).

The spectrum of more commonly known skin immune responses is represented by contact dermatitis and atopy. Contact dermatitis, a pathogenic manifestation of LC activation, is directed by Langerhans cells which phagocytose antigen, migrate to lymph nodes, present antigen, and sensitize T cells for the intense destructive cellular response that occurs at the affected skin site (Dahl, 1996; Leung, 1997). Atopic dermatitis may utilize the Langerhans cell in a similar fashion, but is identified with Th2 cells and is generally associated with high levels of IgE antibody (Dahl, 1996; Leung, 1997).

Transcutaneous immunization with cholera toxin and related bAREs on the other hand is a novel immune response with an absence of superficial and microscopic post-immunization skin findings (i.e., non-inflamed skin) shown by the absence of lymphocyte infiltration 24, 48 and 120 hours after immunization with cholera toxin. This indicates that Langerhans cells "comprise all of the accessory cell activity that is present in uninflammed epidermis, and in the current paradigm are essential for the initiation and propagation of immune responses directed against epicutaneously applied antigens" (Udey, 1997). The uniqueness of the transcutaneous immune response here is also indicated by the both high levels of antigen-specific IgG antibody, and the type of antibody produced (e.g., IgM, IgG1, IgG2a, IgG2b, IgG3 and IgA) and the absence of anti-CT IgE antibody.

Thus, we have found that bacterial-derived toxins applied to the surface of the skin can activate Langerhans cells or other antigen presenting cells, and induce a potent immune response manifested as high levels of antigen-specific circulating IgG antibodies. Such adjuvants may be used in transcutaneous immunization to enhance the IgG antibody response to proteins not otherwise immunogenic by themselves when placed on the skin.

Transcutaneous targeting of Langerhans cells may also be used to deactivate their antigen presenting function, thereby preventing immunization or sensitization. Techniques to deactivate Langerhans cells include, for example, the use of interleukin-10 (Peguet-Navarro et al., 1995), monoclonal antibody to interleukin-1β (Enk et al., 1993), or depletion via superantigens such as through staphylococcal enterotoxin-A (SEA) induced epidermal Langerhans cell depletion (Shankar et al., 1996).

Transcutaneous immunization may be induced via the ganglioside GM1 binding activity of CT, LT or subunits such as CTB (Craig and Cuatrecasas, 1975). Ganglioside GM1 is a ubiquitous cell membrane glycolipid found in all mammalian cells (Plotkin and Mortimer, 1994). When the pentameric CT B subunit binds to the cell surface a hydrophilic pore is formed which allows the A subunit to penetrate across the lipid bilayer (Ribi et al., 1988).

We have shown that transcutaneous immunization by CT or CTB may require ganglioside GM1 binding activity. When mice were transcutaneously immunized with CT, CTA and CTB, only CT and CTB resulted in an immune response. CTA contains the ADP-ribosylating exotoxin activity but only CT and CTB containing the binding activity were able to induce an immune response indicating that the B subunit was necessary and sufficient to immunize through the skin. We conclude that the Langerhans cell or another antigen presenting cell may be activated by CTB binding to its cell surface.

ANTIGEN

Antigen of the invention may be expressed by recombinant means, preferably as a fusion with an affinity or epitope tag (Summers and Smith, 1987; Goeddel, 1990; Ausubel et al., 1996); chemical synthesis of an oligopeptide, either free or conjugated to carrier proteins, may be used to obtain antigen of the invention (Bodanszky, 1993; Wisdom, 1994). Oligopeptides are considered a type of polypeptide.

Oligopeptide lengths of 6 residues to 20 residues are preferred. Polypeptides may also by synthesized as branched structures such as those disclosed in U.S. Pat. Nos. 5,229,490 and 5,390,111. Antigenic polypeptides include, for example, synthetic or recombinant B-cell and T-cell epitopes, universal T-cell epitopes, and mixed T-cell epitopes from one organism or disease and B-cell epitopes from another.

Antigen obtained through recombinant means or peptide synthesis, as well as antigen of the invention obtained from natural sources or extracts, may be purified by means of the antigen's physical and chemical characteristics, preferably by fractionation or chromatography (Janson and Ryden, 1989; Deutscher, 1990; Scopes, 1993).

A multivalent antigen formulation may be used to induce an immune response to more than one antigen at the same time. Conjugates may be used to induce an immune response to multiple antigens, to boost the immune response, or both. Additionally, toxins may be boosted by the use of toxoids, or toxoids boosted by the use of toxins. Transcutaneous immunization may be used to boost responses induced initially by other routes of immunization such as by injection, or the oral or intranasal routes.

Antigen includes, for example, toxins, toxoids, subunits thereof, or combinations thereof (e.g., cholera toxin, tetanus toxoid).

Antigen may be solubilized in water, a solvent such as methanol, or a buffer. Suitable buffers include, but are not limited to, phosphate buffered saline $Ca^{++}/Mg^{++}$ free (PBS), normal saline (150 mM NaCl in water), and Tris buffer. Antigen not soluble in neutral buffer can be solubilized in 10 mM acetic acid and then diluted to the desired volume with a neutral buffer such as PBS. In the case of antigen soluble only at acid pH, acetate-PBS at acid pH may be used as a diluent after solubilization in dilute acetic acid. Glycerol may be a suitable non-aqueous buffer for use in the present invention.

If an antigen such as, for example, hepatitis A virus, is not soluble per se, the antigen may be present in the formulation in a suspension or even as an aggregate.

Hydrophobic antigen can be solubilized in a detergent, for example a polypeptide containing a membrane-spanning domain. Furthermore, for formulations containing liposomes, an antigen in a detergent solution (e.g., a cell membrane extract) may be mixed with lipids, and liposomes then may be formed by removal of the detergent by dilution, dialysis, or column chromatography. Certain antigens such as, for example, those from a virus (e.g., hepatitis A) need not be soluble per se, but can be incorporated directly into a liposome in the form of a virosome (Morein and Simons, 1985).

Plotkin and Mortimer (1994) provide antigens which can be used to vaccinate animals or humans to induce an immune response specific for particular pathogens, as well as methods of preparing antigen, determining a suitable dose of antigen, assaying for induction of an immune response, and treating infection by a pathogen (e.g., bacterium, virus, fungus, or parasite).

Bacteria include, for example: anthrax, campylobacter, cholera, diphtheria, enterotoxigenic *E. coli,* giardia, gonococcus, *Helicobacter pylori* (Lee and Chen, 1994), *Hemophilus influenza* B, *Hemophilus influenza* non-typable, meningococcus, pertussis, pneumococcus, salmonella, shigella, *Streptococcus B,* group A *Streptococcus,* tetanus, *Vibrio cholerae,* yersinia, *Staphylococcus, Pseudomonas* species and *Clostridia* species.

Viruses include, for example: adenovirus, dengue serotypes 1 to 4 (Delenda et al., 1994; Fonseca et al., 1994; Smucny et al., 1995), ebola (Jahrling et al., 1996), enterovirus, hepatitis serotypes A to E (Blum, 1995; Katkov, 1996; Lieberman and Greenberg, 1996; Mast, 1996; Shafara et al., 1995; Smedila et al., 1994; U.S. Pat. Nos. 5,314,808 and 5,436,126), herpes simplex virus 1 or 2, human immunodeficiency virus (Deprez et al., 1996), influenza, Japanese equine encephalitis, measles, Norwalk, papilloma virus, parvovirus B19, polio, rabies, rotavirus, rubella, rubeola, vaccinia, vaccinia constructs containing genes coding for other antigens such as malaria antigens, varicella, and yellow fever.

Parasites include, for example: *Entamoeba histolytica* (Zhang et al., 1995); *Plasmodium* (Bathurst et al., 1993; Chang et al., 1989, 1992, 1994; Fries et al., 1992a, 1992b; Herrington et al., 1991; Khusmith et al., 1991; Malik et al., 1991; Migliorini et al., 1993; Pessi et al., 1991; Tam, 1988; Vreden et al., 1991; White et al., 1993; Wiesmueller et al., 1991), *Leishmania* (Frankenburg et al., 1996), Toxoplasmosis, and the Helminths.

Antigens may also comprise those used in biological warfare such as ricin, for which protection can be achieved via antibodies.

ADJUVANT

The formulation also contains an adjuvant, although a single molecule may contain both adjuvant and antigen properties (e.g., cholera toxin) (Elson and Dertzbaugh, 1994). Adjuvants are substances that are used to specifically or non-specifically potentiate an antigen-specific immune response. Usually, the adjuvant and the formulation are mixed prior to presentation of the antigen but, alternatively, they may be separately presented within a short interval of time.

Adjuvants include, for example, an oil emulsion (e.g., complete or incomplete Freund's adjuvant), a chemokine (e.g., defensins 1 or 2, RANTES, MIP1-$\alpha$, MIP-2, interleukin-8) or a cytokine (e.g., interleukin-1$\beta$, -2, -6, -10 or -12; $\gamma$-interferon; tumor necrosis factor-$\alpha$; or granulocyte-monocyte-colony stimulating factor) (reviewed in Nohria and Rubin, 1994), a muramyl dipeptide derivative (e.g., murabutide, threonyl-MDP or muramyl tripeptide), a heat shock protein or a derivative, a derivative of *Leishmania major* LeIF (Skeiky et al., 1995), cholera toxin or cholera toxin B, a lipopolysaccharide (LPS) derivative (e.g., lipid A or monophosphoryl lipid A), or superantigen (Saloga et al., 1996). Also, see Richards et al. (1995) for adjuvants useful in immunization.

An adjuvant may be chosen to preferentially induce antibody or cellular effectors, specific antibody isotypes (e.g., IgM, IgD, IgA1, IgA2, secretory IgA, IgE, IgG1, IgG2, IgG3, and/or IgG4), or specific T-cell subsets (e.g., CTL, Th1, Th2 and/or $T_{DTH}$) (Glenn et al., 1995).

Cholera toxin is a bacterial exotoxin from the family of ADP-ribsoylating exotoxins (referred to as bAREs). Most bAREs are organized as A:B dimer with a binding B subunit and an A subunit containing the ADP-ribosyltransferase. Such toxins include diphtheria toxin, *Pseudomonas* exotoxin A, cholera toxin (CT), E. *coli* heat-labile enterotoxin (LT), pertussis toxin, C. *botulinum* toxin C2, C. *botulinum* toxin C3, *C. limosum* exoenzyme, *B. cereus* exoenzyme, *Pseudomonas* exotoxin S, *Staphylococcus aureus* EDIN, and *B. sphaericus* toxin.

Cholera toxin is an example of a bARE that is organized with A and B subunits. The B subunit is the binding subunit and consists of a B-subunit pentamer which is non-covalently bound to the A subunit. The B-subunit pentamer is arranged in a symmetrical doughnut-shaped structure that binds to $GM_1$-ganglioside on the target cell. The A subunit serves to ADP ribosylate the alpha subunit of a subset of the hetero trimeric GTP proteins (G proteins) including the Gs protein which results in the elevated intracellular levels of cyclic AMP. This stimulates release of ions and fluid from intestinal cells in the case of cholera.

Cholera toxin (CT) and its B subunit (CTB) have adjuvant properties when used as either an intramuscular or oral immunogen (Elson and Dertzbaugh, 1994; Trach et al., 1997). Another antigen, heat-labile enterotoxin from *E. coli* (LT) is 80% homologous at the amino acid level with CT and possesses similar binding properties; it also appears to bind the $GM_1$-ganglioside receptor in the gut and has similar ADP-ribosylating exotoxin activities. Another bARE, *Pseudomonas* exotoxin A (ETA), binds to the $\alpha_2$-macroglobulin receptor-low density lipoprotein receptor-related protein (Kounnas et al., 1992). bAREs are reviewed by Krueger and Barbieri (1995).

The toxicity of CT by oral, nasal, and intramuscular routes limits the dose that can be used as an adjuvant. In a comparative trial of CT injected intramuscularly, extensive swelling at the site of injection was elicited. By contrast, equivalent or greater doses of CT placed on the skin caused no toxicity.

The examples below show that cholera toxin (CT), its B subunit (CTB), *E. coli* heat-labile enterotoxin (LT), and pertussis toxin are potent adjuvants for transcutaneous immunization, inducing high levels of IgG antibodies but not IgE antibodies. Also shown is that CTB without CT can also induce high levels of IgG antibodies. Thus, both bAREs and a derivative thereof can effectively immunize when epicutaneouly applied to the skin in a simple solution. Furthermore, these examples demonstrate that CT, CTB and bAREs can act as both adjuvant and antigen.

When an adjuvant such as CT is mixed with BSA, a protein not usually immunogenic when applied to the skin, anti-BSA antibodies are induced. An immune response to diphtheria toxoid was induced using pertussis toxin as adjuvant, but not with diphtheria toxoid alone. Thus, bAREs can act as adjuvants for non-immunogenic proteins in an transcutaneous immunization system.

Other proteins may also act as both adjuvant and antigen. For example FLUZONE (Lederle), the split virion influenza A and B vaccine contains neuraminidase and hemagglutinin which are highly immunogenic, confers protection and may effectively immunize through the skin acting as its own adjuvant and antigen. Toxoids such as diphtheria toxoid which has been toxoided using formalin, pertussis toxoid which has been toxoided using hydrogen peroxide, or mutant toxins such as cholera or heat labile enterotoxin from E. *coli* which have been toxoided using genetic techniques to destroy the ribosyl transferase activity, may continue to harbor adjuvant qualities and act as both antigen and adjuvant.

Protection against the life-threatening infections diphtheria, pertussis, and tetanus (DPT) can be achieved by inducing high levels of circulating anti-toxin antibodies. Pertussis may be an exception in that some investigators feel that antibodies directed to other portions of the invading organism are necessary for protection, although this is controversial (see Schneerson et al., 1996) and most new generation acellular pertussis vaccines have PT as a component of the vaccine (Krueger and Barbieri, 1995). The pathologies in the diseases caused by DPT are directly related to the effects of their toxins and anti-toxin antibodies most certainly play a role in protection (Schneerson et al., 1996).

In general, toxins can be chemically inactivated to form toxoids which are less toxic but remain immunogenic. We envision that the transcutaneous immunization system using toxin-based immunogens and adjuvants can achieve anti-toxin levels adequate for protection against these diseases. The anti-toxin antibodies may be induced through immunization with the toxins, or genetically-detoxified toxoids themselves, or with toxoids and adjuvants such as CT or by the toxoids alone. Genetically toxoided toxins which have altered ADP-ribosylating exotoxin activity, but not binding activity, are envisioned to be especially useful as non-toxic activators of antigen presenting cells used in transcutaneous immunization.

We envision that CT can also act as an adjuvant to induce antigen-specific CTLs through transcutaneous immunization (see Bowen et al., 1994; Porgador et al., 1997 for the use of CT as an adjuvant in oral immunization).

The bARE adjuvant may be chemically conjugated to other antigens including, for example, carbohydrates, polypeptides, glycolipids, and glycoprotein antigens. Chemical conjugation with toxins, their subunits, or toxoids with these antigens would be expected to enhance the immune response to these antigens when applied epicutaneously.

To overcome the problem of the toxicity of the toxins, (e.g., diphtheria toxin is known to be so toxic that one molecule can kill a cell) and to overcome the difficulty of working with such potent toxins as tetanus, several workers have taken a recombinant approach to producing genetically produced toxoids. This is based on inactivating the catalytic activity of the ADP-ribosyl transferase by genetic deletion. These toxins retain the binding capabilities, but lack the toxicity, of the natural toxins. This approach is described by Burnette et al. (1994), Rappuoli et al. (1995), and Rappuoli et al. (1996). Such genetically toxoided exotoxins could be useful for transcutaneous immunization system in that they would not create a safety concern as the toxoids would not be considered toxic. They may act as both antigens and adjuvants, enhancing the immune response to themselves or added antigens. Additionally, several techniques exist to chemically toxoid toxins which can address the same problem (Schneerson et al., 1996). Alternatively, fragments of the toxin or toxoids may be used such as the C fragment of Tetanus. These techniques could be important for certain applications, especially pediatric applications, in which ingested toxins (e.g., diphtheria toxin) might possibly create adverse reactions.

Optionally, an activator of Langerhans cells may be used as an adjuvant. Examples of such activators include: inducers of heat shock protein; contact sensitizers (e.g., trinitrochlorobenzene, dinitrofluorobenzene, nitrogen mustard, pentadecylcatechol); toxins (e.g, Shiga toxin, Staph enterotoxin B); lipopolysaccharides, lipid A, or derivatives thereof; bacterial DNA (Stacey et al., 1996); cytokines (e.g., tumor necrosis factor-$\alpha$, interleukin-1$\beta$, -10, -12); and chemokines (e.g., defensins 1 or 2, RANTES, MIP-1$\alpha$, MIP-2, interleukin-8).

A combination of different adjuvants may be used in the present invention. For example, a combination of bacterial DNA containing CpG nucleotide sequences and an ADP-ribosylating exotoxin could be used to direct the T-helper response to antigens administered transcutaneously. Thus, Th1 or Th2 like responses to CT-adjuvanted antigens could be switched by the use of nonmethylated CpG bacterial DNA, or other proteins such as LeIF or calcium channel blockers.

CpGs are among a class of structures which have patterns allowing the immune system to recognize their pathogenic origins to stimulate the innate immune response leading to adaptive immune responses. (Medzhitov and Janeway, Curr. Opin. Immunol., 9:4-9, 1997). These structures are called pathogen-associated molecular patterns (PAMPs) and include lipopolysaccharides, teichoic acids, unmethylated CpG motifs, double stranded RNA and mannins.

PAMPs induce endogenous signals that can mediate the inflammatory response, act as costimulators of T-cell function and control the effector function. The ability of PAMPs to induce these responses play a role in their potential as adjuvants and their targets are APCs such as macrophages and dendritic cells. The antigen presenting cells of the skin could likewise be stimulated by PAMPs transmitted through the skin. For example, Langerhans cells, a type of dendritic cell, could be activated by a PAMP in solution on the skin with a transcutaneously poorly immunogenic molecule and be induced to migrate and present this poorly immunogenic molecule to T-cells in the lymph node, inducing an antibody response to the poorly immunogenic molecule. PAMPs could also be used in conjunction with other skin adjuvants such as cholera toixn to induce different costimulatory molecules and control different effector functions to guide the immune response, for example from a Th2 to a Th1 response.

If an immunizing antigen has sufficient Langerhans cell activating capabilities then a separate adjuvant may not be required, as in the case of CT which is both antigen and adjuvant. It is envisioned that whole cell preparations, live viruses, attenuated viruses, DNA plasmids, and bacterial DNA could be sufficient to immunize transcutaneously. It may be possible to use low concentrations of contact sensitizers or other activators of Langerhans cells to induce an immune response without inducing skin lesions.

## LIPOSOMES AND THEIR PREPARATION

Liposomes are closed vesicles surrounding an internal aqueous space. The internal compartment is separated from the external medium by a lipid bilayer composed of discrete lipid molecules. In the present invention, antigen may be delivered through intact skin to specialized cells of the immune system, whereby an antigen-specific immune response is induced. Transcutaneous immunization may be achieved by using liposomes; however, as shown in the examples, liposomes are not required to elicit an antigen-specific immune response.

Liposomes may be prepared using a variety of techniques and membrane lipids (reviewed in Gregoriadis, 1993). Liposomes may be pre-formed and then mixed with antigen. The antigen may be dissolved or suspended, and then added to (a) the pre-formed liposomes in a lyophilized state, (b) dried lipids as a swelling solution or suspension, or (c) the solution of lipids used to form liposomes. They may also be formed from lipids extracted from the stratum corneum including, for example, ceramide and cholesterol derivatives (Wertz, 1992).

Chloroform is a preferred solvent for lipids, but it may deteriorate upon storage. Therefore, at one-to three-month intervals, chloroform is redistilled prior to its use as the solvent in forming liposomes. After distillation, 0.7% ethanol can be added as a preservative. Ethanol and methanol are other suitable solvents.

The lipid solution used to form liposomes is placed in a round-bottomed flask. Pear-shaped boiling flasks are preferred, particularly those flasks sold by Lurex Scientific (Vineland, NJ, cat. no. JM-5490). The volume of the flask should be more than ten times greater than the volume of the anticipated aqueous suspension of liposomes to allow for proper agitation during liposome formation.

Using a rotary evaporator, solvent is removed at 37°C under negative pressure for 10 minutes with a filter aspirator attached to a water faucet. The flask is further dried under low vacuum (i.e., less than 50 mm Hg) for 1 hour in a dessicator.

To encapsulate antigen into liposomes, an aqueous solution containing antigen may be added to lyophilized liposome lipids in a volume that results in a concentration of approximately 200 mM with respect to liposome lipid, and shaken or vortexed until all the dried liposome lipids are wet. The liposome-antigen mixture may then be incubated for 18 hours to 72 hours at 4°C. The liposome-antigen formulation may be used immediately or stored for several years. It is preferred to employ such a formulation directly in the transcutaneous immunization system without removing unencapsulated antigen. Techniques such as bath sonication may be employed to decrease the size of liposomes, which may augment transcutaneous immunization.

Liposomes may be formed as described above but without addition of antigen to the aqueous solution. Antigen may then be added to the pre-formed liposomes and, therefore, antigen would be in solution and/or associated with, but not encapsulated by, the liposomes. This process of making a liposome-containing formulation is preferred because of its simplicity. Techniques such as bath sonication may be employed to alter the size and/or lamellarity of the liposomes to enhance immunization.

Although not required to practice the present invention, hydration of the stratum corneum may be enhanced by adding liposomes to the formulation. Liposomes have been used as carriers with adjuvants to enhance the immune response to antigens mixed with, encapsulated in, attached to, or associated with liposomes.

TRANSCUTANEOUS DELIVERY OF ANTIGEN

Efficient immunization can be achieved with the present invention because transcutaneous delivery of antigen may target the Langerhans cell. These cells are found in abundance in the skin and are efficient antigen presenting cells leading to T-cell memory and potent immune responses (Udey, 1997). Because of the presence of large numbers of Langerhans cells in the skin, the efficiency of transcutaneous delivery may be related to the surface area exposed to antigen and adjuvant. In fact, the reason that transcutaneous immunization is so effective may be that it targets a larger number of these efficient antigen presenting cells than intramuscular immunization. However, even a small number of Langerhans cells or dendritic cells may be sufficient for immunization.

We envision the present invention will enhance access to immunization, while inducing a potent immune response. Because transcutaneous immunization does not involve penetration of the skin and the complications and difficulties thereof, the requirements of trained personnel, sterile technique, and sterile equipment are reduced. Furthermore, the barriers to immunization at multiple sites or to multiple immunizations are diminished. Immunization by a single application of the formulation is also envisioned.

Immunization may be achieved using epicutaneous application of a simple solution of antigen and adjuvant impregnated in gauze under an occlusive patch, or by using other patch technologies; creams, immersion, ointments and sprays are other possible methods of application. The immunization could be given by untrained personnel, and is amenable to self-application. Large-scale field immunization could occur given the easy accessibility to immunization. Additionally, a simple immunization procedure would improve access to immunization by pediatric patients and the elderly, and populations in Third World countries.

Similarly, animals could be immunized using the present invention. Application to anatomical sites such as the ear, underbelly, paws, conjunctiva, intertriginous regions, or anal region, or via dipping or immersion could be employed.

For previous vaccines, their formulations were injected through the skin with needles. Injection of vaccines using needles carries certain drawbacks including the pain associated with injections, the need for sterile needles and syringes, trained medical personnel to administer the vaccine, discomfort from the injection, and potential complications brought about by puncturing the skin with the needle. Immunization through the skin without the use of needles (i.e., transcutaneous immunization) represents a major advance for vaccine delivery by avoiding the aforementioned drawbacks.

The transcutaneous delivery system of the invention is also not concerned with penetration of intact skin by sound or electrical energy. Such a system that uses an electrical field to induce dielectric breakdown of the stratum corneum is disclosed in U.S. Pat. No. 5,464,386.

Moreover, transcutaneous immunization may be superior to immunization using needles as more immune cells would be targeted by the use of several locations targeting large surface areas of skin. A therapeutically effective amount of antigen sufficient to induce an immune response may be delivered transcutaneously either at a single cutaneous location, or over an area of intact skin covering multiple draining lymph node fields (e.g., cervical, axillary, inguinal, epitrochelear, popliteal, those of the abdomen and thorax). Such locations close to numerous different lymphatic nodes at locations all over the body will provide a more widespread stimulus to the immune system than when a small amount of antigen is injected at a single location by intradermal subcutaneous or intramuscular injection.

Antigen passing through or into the skin may encounter antigen presenting cells which process the antigen in a way that induces an immune response. Multiple immunization sites may recruit a greater number of antigen presenting cells and the larger population of antigen presenting cells that were recruited would result in greater induction of the immune response. Transcutaneous immunization may allow application in close proximity to a lymph node draining site and thereby improve efficiency or potency of immunization. It is conceivable that absorption through the skin may deliver antigen to phagocytic cells of the skin such as, for example, dermal dendritic cells, macrophages, and other skin antigen presenting cells; antigen may also be delivered to phagocytic cells of the liver, spleen, and bone marrow that are known to serve as the antigen presenting cells through the blood stream or lymphatic system. The result would be widespread distribution of antigen to antigen presenting cells to a degree that is rarely, if ever achieved, by current immunization practices.

The transcutaneous immunization system may be applied directly to the skin and allowed to air dry; rubbed into the skin or scalp; held in place with a dressing, patch, or absorbent material; otherwise held by a device such as a stocking, slipper, glove, or shirt; or sprayed onto the skin to maximize contact with the skin. The formulation may be applied in an absorbant dressing or gauze. The formulation may be covered with an occlusive dressing such as, for example, in an emulsion of antigen solution and AQUAPHOR (petrolatum, mineral oil, mineral wax, wool wax, panthenol, bisabol, and glycerin from Beiersdorf), plastic film, impregnated polymer, COMFEEL (Coloplast) or vaseline; or a non-occlusive dressing such as, for example, DUODERM (3M) or OPSITE (Smith & Napheu). An occlusive dressing completely excludes the passage of water. Alternatively, a partially occlusive dressing such as TEGADERM may be applied to provide hydration and may allow longer application of the patch or may prevent maceration of the skin.

The formulation may be applied to single or multiple sites, to single or multiple limbs, or to large surface areas of the skin by complete immersion. The formulation may be applied directly to the skin.

Genetic immunization has been described in U.S. Pat. Nos. 5,589,466 and 5,593,972. The nucleic acid(s) contained in the formulation may encode the antigen, the adjuvant, or both. The nucleic acid may or may not be capable of replication; it may be non-integrating and non-infectious. The nucleic acid may further comprise a regulatory region (e.g., promoter, enhancer, silencer, transcription initiation and termination sites, RNA splice acceptor and donor sites, polyadenylation signal, internal ribosome binding site, translation initiation and termination sites) operably linked to the sequence encoding the antigen or adjuvant. The nucleic acid may be complexed with an agent that promotes transfection such as cationic lipid, calcium phosphate, DEAE-dextran, polybrene-DMSO, or a combination thereof. The nucleic acid may comprise regions derived from viral genomes. Such materials and techniques are described by Kriegler (1990) and Murray (1991).

An immune response may comprise humoral (i.e., antigen-specific antibody) and/or cellular (i.e., antigen-specific lymphocytes such as B cells, CD4+ T cells, CD8+ T cells, CTL, Th1 cells, Th2 cells, and/or $T_{DTH}$ cells) effector arms. Moreover, the immune response may comprise NK cells that mediate antibody-dependent cell-mediated cytotoxicity (ADCC).

The immune response induced by the formulation of the invention may include the elicitation of antigen-specific antibodies and/or cytotoxic lymphocytes (CTL, reviewed in Alving and Wassef, 1994). Antibody can be detected by immunoassay techniques, and the detection of various isotypes (e.g., IgM, IgD, IgA1, IgA2, secretory IgA, IgE, IgG1, IgG2, IgG3, or IgG4) may be expected. An immune response can also be detected by a neutralizing assay.

Antibodies are protective proteins produced by B lymphocytes. They are highly specific, generally targeting one epitope of an antigen. Often, antibodies play a role in protection against disease by specifically reacting with antigens derived from the pathogens causing the disease. Immunization may induce antibodies specific for the immunizing antigen, such as cholera toxin. These antigen-specific antibodies are induced when antigen is delivered through the skin by liposomes.

CTLs are particular protective immune cells produced to protect against infection by a pathogen. They are also highly specific. Immunization may induce CTLs specific for the antigen, such as a synthetic oligopeptide based on a malaria protein, in association with self-major histocompatibility antigen. CTLs induced by immunization with the transcutaneous delivery system may kill pathogen infected cells. Immunization may also produce a memory response as indicated by boosting responses in antibodies and CTLs, lymphocyte proliferation by culture of lymphocytes stimulated with the antigen, and delayed type hypersensitivity responses to intradermal skin challenge of the antigen alone.

It is envisioned that the T-helper response induced by transcutaneous immunization may be manipulated by the use of calcium channel blockers (e.g., nifedipine, verapamil) which suppress the contact hypersensitivity reaction by inhibiting antigen catabolism and subsequent presentation by epidermal Langerhans cells. The transcutaneous application of a calcium channel blocker would be expected to affect surface expression of costimulatory molecules (e.g., B7-related family) and the generation of a subsequent T-helper response. It is also envisioned that addition of the calcium channel blocker may inhibit delayed type hypersensitivity responses and could be used to select an immune response that is predominantly a cellular or a humoral response.

In a viral neutralization assay, serial dilutions of sera are added to host cells which are then observed for infection after challenge with infectious virus. Alternatively, serial dilutions of sera may be incubated with infectious titers of virus prior to inoculation of an animal, and the inoculated animals are then observed for signs of infection.

The transcutaneous immunization system of the invention may be evaluated using challenge models in either animals or humans, which evaluate the ability of immunization with the antigen to protect the subject from disease. Such protection would demonstrate an antigen-specific immune response. In lieu of challenge, achieving anti-diphtheria antibody titers of 5 IU/ml or greater is generally assumed to indicate optimum protection and serves as a surrogate marker for protection (Plotkin and Mortimer, 1994).

Furthermore, the *Plasmodium faciparum* challenge model may be used as to induce an antigen-specific immune response in humans. Human volunteers may be immunized using the transcutaneous immunization system containing oligopeptides or proteins (polypeptides) derived from the malaria parasite, and then exposed to malaria experimentally or in the natural setting. The *Plasmodium yoelii* mouse malaria challenge model may be used to evaluate protection in the mouse against malaria (Wang et al, 1995).

Alving et al (1986) injected liposomes comprising lipid A as an adjuvant for inducing an immune response to cholera toxin (CT) in rabbits and to a synthetic protein consisting of a malaria oligopeptide containing four tetra-peptides (Asn-Ala-Asn-Pro) conjugated to BSA. The authors found that the immune response to cholera toxin or to the synthetic malaria protein was markedly enhanced by encapsulating the antigen within the liposomes containing lipid A, compared to similar liposomes lacking lipid A. Several antigens derived either from the circumsporozoite protein (CSP) or from merozoite surface proteins of *Plasmodium falciparum* have been encapsulated in liposomes containing lipid A. All of the malaria antigens that have been encapsulated in liposomes containing lipid A have been shown to induce humoral effectors (i.e., antigen-specific antibodies), and some have been shown to induce cell-mediated responses as well. Generation of an immune response and immunoprotection in an animal vaccinated with a malaria antigen may be assayed by immunofluorescence to whole, fixed malaria sporozoites or CTLs killing of target cells transfected with CSP.

Mice transcutaneously immunized with cholera toxin can be protected against intranasal challenge with a 20 μg dose of cholera toxin. Mallet et al (personal communication) have found that C57B1/6 mice develop a fatal hemorrhagic pneumonia in response to intranasal challenge with CT. Alternatively, the mice may be challenged with an intraperitoneal dose of CT (Dragunsky et al, 1992). Cholera toxin-specific IgG or IgA antibody may provide protection against cholera toxin challenge (Pierce, 1978; Pierce and Reynolds, 1974).

Similar protective effects could be expected in humans immunized with LT or CT, and challenged with LT-secreting *E. coli* or CT-secreting *Vibrio cholerae,* respectively. Additionally, cross protection has been demonstrated between CT and LT immune subjects and CT and LT mediated disease.

As shown in the examples below, mucosal immunity may be achieved via the trancutaneous route. Mucosal IgG and IgA can be detected in mice immunized with CT transcutaneously. This may be important for protection in diseases where the pathology occurs at mucosal sites such as in LT or CT mediated disease, where entry of the pathogenic organism occurs at a mucosal site, or where mucosal infection is important to pathogenesis.

It would be expected that transcutaneous immunization against diseases such as influenza could be effective either by inducing mucosal immunity or by systemic immunity, or by a combination of immunity such as humoral, cellular or mucosal.

Vaccines may be effective against host effects such as the binding of erythrocytes to vascular endothelium in malaria by inducing anti-sequestrin antibodies.

Protective antibodies such as anti-hepatitis A, B or hepatitis E antibodies may be induced by the transcutaneous route using whole inactivated virus, virus-derived subunits or recombinant products.

Protection against tetanus, diphtheria and other toxin mediated diseases may be conferred by transcutaneously induced anti-toxin antibodies. A tetanus "booster" patch could be envisioned that contained adjuvant such as CT and toxoids such as tetanus and diphtheria, or fragments such as the tetanus C fragment. Boosting could be achieved following primary immunization by injection or transcutaneous immunization with the same or similar antigens. For injectable immunizations that induce immunity but have potential side effects upon boosting, a transcutaneous boost may be preferable. Oral or nasal immunization may conceivably be boosted using the transcutaneous route. Simultaneous use of injectable and transcutaneous immunizations could also be used.

Vaccination has also been used as a treatment for cancer and autoimmune disease. For example, vaccination with a tumor antigen (e.g., prostate specific antigen) may induce an immune response in the form of antibodies, CTLs and lymphocyte proliferation which allows the body's immune system to recognize and kill tumor cells. Targeting dendritic cells, of which Langerhans cells are a specific subset, has been shown to be an important strategy in cancer immunotherapy. Tumor antigens useful for vaccination have been described for melanoma (U.S. Pat. Nos. 5,102,663, 5,141,742, and 5,262,177), prostate carcinoma (U.S. Pat. No. 5,538,866), and lymphoma (U.S. Pat. Nos. 4,816,249, 5,068,177, and 5,227,159). Vaccination with T-cell receptor oligopeptide may induce an immune response that halts progression of autoimmune disease (U.S. Pat. Nos. 5,612,035 and 5,614,192; Antel et al, 1996; Vandenbark et al, 1996). U.S. Pat. No. 5,552,300 also describes antigens suitable for treating autoimmune disease.

The following is meant to be illustrative of the present invention; however, the practice of the invention is not limited or restricted in any way by the examples.

EXAMPLES

Immunization Procedure

BALB/c mice of 6 to 8 weeks were shaved with a #40 clipper. This shaving could be done without any signs of trauma to the skin. The shaving was done from the mid-thorax to just below the nape of the neck. The mice were then allowed to rest for 24 hours. Prior to this the mice had been ear-tagged for identification, and pre-bled to obtain a sample of pre-immune serum. Mice were also transcutaneously immunized without shaving by applying up to 50 μl of immunizing solution to each ear.

The mice were then immunized in the following way. Mice were anesthetized with 0.03-0.06 ml of a 20 mg/ml solution of xylazine and 0.5 ml of 100 mg/ml ketamine; mice were immobilized by this dose of anesthesia for approximately one hour. The mice were placed ventral side down on a warming blanket.

The immunizing solution was then placed on the dorsal shaved skin of a mouse in the following manner: a 1.2 cm x 1.6 cm stencil made of polystyrene was laid gently on the back and a saline-wetted sterile gauze was used to partially wet the skin (this allowed even application of the immunizing solution), the immunizing solution was then applied with a pipet to the area circumscribed by the stencil to yield a 2 cm$^2$ patch of immunizing solution. Alternatively, a fixed volume of immunizing solution was evenly applied to the shaved area or the ear. Care was used not to scrape or rub the skin with the pipet tip. The immunizing solution was spread around the area to be covered with the smooth side of the pipet tip.

The immunizing solution (between about 100 μl to about 200 μl) was left on the back of the mouse for 60 to 180 minutes. At the end of 60 minutes, the mouse was held gently by the nape of the neck and the tail under a copious stream of lukewarm tap water, and washed for 10 seconds. The mouse was then gently patted dry with a piece of sterile gauze and a second washing was performed for 10 seconds; the mouse was then patted dry a second time and left in the cage. The mice appeared to exhibit no adverse effects from the anesthesia, immunization, washing procedure, or toxicity from the exotoxins. No skin irritation, swelling or redness was seen after the immunization and the mice appeared to thrive. Immunization using the ear was performed as described above except that fur was not removed prior to immunization.

Antigen

The following antigens were used for immunization and ELISA, and were mixed using sterile PBS or normal saline. Cholera toxin or CT (List Biologicals, Cat #101B, lot #10149CB), CT B subunit (List Biologicals, Cat #BT01, lot #CVXG-14E), CT A subunit (List Biologicals, Cat #102A, lot #CVXA-17B), CT A subunit (Calbiochem, Cat #608562); pertussis toxin, salt-free (List Biologicals, lot #181120a); tetanus toxoid (List Biologicals, lots #1913a and #1915a); *Pseudomonas* exotoxin A (List Biologicals, lot #ETA25a); diphtheria toxoid (List Biologicals, lot #15151); heat-labile enterotoxin from E. *coli* (Sigma, lot #9640625); bovine serum albumin or BSA (Sigma, Cat #3A-4503, lot #31F-0116); and *Hemophilus influenza* B conjugate (Connaught, lot#6J81401).

ELISA - IgG(H+L)

Antibodies specific for CT, LT, ETA, pertussis toxin, diphtheria toxoid, tetanus toxoid, *Hemophilus influenza* B conjugate, influenza, sequestrin, and BSA were determined using ELISA in a technique similar to Glenn et al (1995). All antigens were dissolved in sterile saline at a concentration of 2 μg/ml. Fifty microliters of this solution (0.1 μg) per well was put on IMMULON-2 polystyrene plates (Dynatech Laboratories, Chantilly, VA) and incubated at room temperature overnight. The plates were then blocked with a 0.5% casein/0.05% Tween 20 blocking buffer solution for one hour. Sera was diluted with 0.5% casein/0.05% Tween 20 diluent; dilution series were done in columns on the plate. Incubation was for 2 hours at room temperature.

The plates were then washed in a PBS-0.05% Tween 20 wash solution four times, and goat anti-mouse IgG (H+L) horseradish peroxidase (HRP)-linked (Bio-Rad Laboratories, Richmond, CA, Cat #170-6516) secondary antibody was diluted in casein diluent at a dilution of 1/500 and left on the plates for one hour at room temperature. The plates were then washed four times in the PBS-Tween wash solution. One hundred microliters of 2,2'-azino-di(3-ethyl-benzthiazolone) sulphonic acid substrate (Kirkegaard and Perry) were added to each well and the plates were read at 405 nm after 20-40 minutes of development. Results are reported as the geometric mean of individual sera and standard error of the mean of ELISA units (the serum dilution at which the absorbance in equal to 1.0) or as individual antibody responses in ELISA units.

ELISA - IgG(γ), IgM(μ) and IgA(α)

IgG(γ), IgM(μ) and IgA(α) anti-CT antibody levels were determined using ELISA with a technique similar to Glenn et al (1995). CT was dissolved in sterile saline at a concentration of 2 μg/ml. Fifty microliters of this solution (0.1 μg) per well were put on IMMULON-2 polystyrene plates (Dynatech Laboratories, Chantilly, VA) and incubated at room temperature overnight. The plates were then blocked with a 0.5% casein-Tween 20 blocking buffer solution for one hour. Sera was diluted and casein diluent and serial dilutions were done on the plate. This was incubated for two hours at room temperature.

The plates were then washed in a PBS-Tween wash solution four times and goat anti-mouse IgG(γ) HRP-linked (Bio-Rad Laboratories, Richmond, CA, Cat #172-1038), goat anti-mouse IgM(μ) HRP-linked (BioRad Laboratories, Richmond, CA, Cat #172-1030), or goat anti-mouse IgA HRP-linked (Sigma, St. Louis, MO, Cat #1158985) secondary antibody was diluted in casein diluent in a dilution of 1/1000 and left on the plates for one hour at room temperature. The plates were then washed four times in a PBS-Tween wash solution. One hundred microliters of 2,2'-azino-di(3-ethyl benzthiazolone) sulphonic acid substrate from (Kirkegaard and Perry, Gaithersburg, MD) were added to the wells and the plates were read at 405 nm. Results are reported as the geometric mean of individual sera and standard error of the mean of ELISA units (the serum dilution at which the absorbance in equal to 1.0).

ELISA - IgG Subclass

Antigen-specific IgG (IgG1, IgG2a, IgG2b, and IgG3) subclass antibody against CT, LT, ETA, and BSA was performed as described by Glenn et al (1995). The solid phase ELISA was performed in IMMULON-2 polystyrene plates (Dynatech Laboratories, Chantilly, VA). Wells were incubated with the respective antigens in saline overnight (0.1 μg/ 50 μl) and blocked with 0.5% casein-Tween 20. Individual mouse sera diluted in 0.5% casein were serially diluted, and incubated at room temperature for four hours. Secondary antibody consisted of horseradish peroxidase-conjugated goat anti-mouse isotype-specific antibody (IgG1, IgG2a, IgG2b, IgG3, The Binding Site, San Diego, CA). A standard curve for each subclass was determined using mouse myeloma IgG1, IgG2a, IgG2b, and IgG3 (The Binding Site, San Diego, CA). Standard wells were coated with goat anti-mouse IgG(H+L) (Bio-Rad Laboratories, Richmond, CA, Cat #172-1054) to capture the myeloma IgG subclass standards which were added in serial dilutions. The myeloma IgG subclass was also detected using the peroxidase-conjugated goat anti-mouse subclass-specific antibody. Both the test sera and myeloma standards were detected using 2,2'-azino-di(3-ethyl-benzthiazolone) sulphonic acid (Kirkegaard and Perry, Gaithersburg, MD) as substrate. Absorbances were read at 405 nm. Individual antigen specific subclasses were quantitated using the values from the linear titration curve computed against the myeloma standard curve and reported as μg/ml.

ELISA - IgE

Antigen-specific IgE antibody quantitation was performed using a protocol from Pharmingen Technical Protocols, page 541 of the Research Products Catalog, 1996-1997 (Pharmingen, San Diego, CA). Fifty microliters of 2 μg/ml purified anti-mouse IgE capture mAb (Pharmingen, Cat# 02111D) in 0.1 M NaHCO$_3$ (pH 8.2) were added to IMMUNO plates(Nunc, Cat #12-565-136). Plates were incubated overnight at room temperature, washed three times with PBS-Tween 20, blocked with 3% BSA in PBS for two hours, and washed three times with PBS-Tween. Sera were diluted in 1% BSA in PBS, added at dilutions of 1/100, and diluted serially down the columns (e.g., 1/100, 1/200, et cetera). Purified mouse IgE standards (Pharmingen, Cat # 0312D) were added with a starting dilution of 0.25 μg/ml and serially diluted down the columns. Plates were incubated for two hours and washed five times with PBS-Tween.
Biotinylated anti-mouse IgE mAB (Pharmingen, Cat #02122D) to 2 μg/ml in 1% BSA in PBS, incubated for 45 minutes and washed five times with PBS-Tween. Avidin-peroxidase (Sigma A3151, 1:400 of 1 mg/ml solution) was added for 30 min and plates were washed six times with PBS-Tween. Both the test sera and IgE standards were detected using 2,2'-azino-di(3-ethylbenzthiazolone) sulphonic acid (Kirkegaard and Perry, Gaithersburg, MD) as substrate. Absorbances were read at 405 nm. Individual antigen specific subclasses were quantitated using the values from the linear titration curve computed against the IgE standard curve and reported as μg/ml.

Liposome Preparation

Where liposomes were included in the formulation for transcutaneous immunization, multilamellar liposomes composed of dimyristoyl phosphatidyl choline, dimyristoyl phosphatidyl glycerol, cholesterol were prepared according to Alving et al (1993). Dimyristoyl phosphatidylcholine, dimyristoyl phosphatidylglycerol, and cholesterol were purchased from Avanti Polar Lipids Inc. (Alabaster, AL). Stock solutions of the lipids in chloroform were removed from -20°C freezer where they were stored.
The lipids were mixed in a molar ratio of 0.9:0.1:0.75 dimyristoyl phosphatidyl choline, dimyristoyl phosphatidyl glycerol, and cholesterol in a pear shaped flask. Using a rotary evaporator, the solvent was removed at 37°C under negative pressure for 10 minutes. The flask was further dried under low vacuum for two hours in a dessicator to remove residual solvent. The liposomes were swollen at 37 mM phospholipid using sterile water, lyophilized and stored at -20°C. These liposomes were mixed in their lyophilized state with normal saline (pH 7.0) to achieve a designated phospholipid concentration in the saline. Alternatively, the dried lipids were swollen to make liposomes with normal saline (pH 7.0) and were not lyophilized.

Example 1

BALB/c mice at 6 to 8 weeks of age were immunized transcutaneously as described above for "Immunization Procedure", in groups of five mice. The mice were immunized using 100 μl of immunization solution which was prepared as follows: liposomes prepared as described above for "Liposome Preparation" were mixed with saline to form the liposomes. The pre-formed liposomes were then diluted in either saline (liposome alone group) or with CT in saline to yield an immunizing solution containing liposomes at 10-150 mM phospholipid with 100 μg of CT per 100 μl of immunizing solution. CT was mixed in saline to make an immunizing solution containing 100 μg of CT per 100 μg of solution for the group receiving CT alone. Solutions were vortexed for 10 seconds prior to immunization.
The mice were immunized transcutaneously at 0 and 3 weeks. Antibody levels were determined using ELISA as described above for "ELISA IgG(H+L)" 3 weeks after the boosting immunization, and compared against pre-immune sera. As shown in Table 1, the level of anti-CT antibodies induced by CT without liposomes was not different from the level of anti-CT antibodies generated using liposomes except in the mice where 150 mM liposomes were used. CT in saline alone was able to immunize mice against CT to produce high antibody titers.

Table 1. Anti-CT antibodies

| Group | ELISA Units | SEM |
|---|---|---|
| CT alone | 27,482 | (16,635-48,051) |
| CT + 150 mM Liposomes | 4,064 | *(2,845-5,072) |
| CT + 100 mM Liposomes | 35,055 | (25,932-49,269) |
| CT + 50 mM Liposomes | 9,168 | (4,283-12,395) |
| CT + 25 mM Liposomes | 18,855 | (12,294-40,374) |
| CT + 10 mM Liposomes | 28,660 | (18,208-31,498) |
| 50 mM Liposomes | 0 | |

* Significantly different from the Group CT alone (P<0.05)

Example 2

BALB/c mice at 6 to 8 weeks of age were immunized transcutaneously as described above for "Immunization Procedure", in groups of five mice. The mice were immunized at 0 and 3 weeks using 100 $\mu$l of immunization solution prepared as follows: BSA was mixed in saline to make an immunizing solution containing 200 $\mu$g of BSA per 100 $\mu$l of saline for the group receiving BSA alone; BSA and CT were mixed in saline to make an immunizing solution containing 200 $\mu$g of BSA and 100 $\mu$g of CT per 100 $\mu$l of saline for the group receiving BSA and CT. Where liposomes were used, the liposomes were prepared as described above for "Liposome Preparation", and were first mixed with saline to form the liposomes. They were then diluted in BSA or BSA and CT in saline to yield an immunizing solution containing liposomes at 50 mM phospholipid with 200 $\mu$g of BSA per 100 $\mu$l of immunizing solution, or 200 $\mu$g BSA + 100 $\mu$g CT per 100 $\mu$l of immunizing solution. Solutions were vortexed for 10 seconds prior to immunization.
The antibodies were determined using ELISA as described above for "ELISA IgG(H+L)" on sera 3 weeks after the second immunization. The results are shown in Table 2. BSA alone, with or without liposomes, was not able to elicit an antibody response. However, the addition of CT stimulated an immune response to BSA. CT acted as a adjuvant for the immune response to BSA, and anti-BSA antibodies of high titer were produced.

Table 2. Anti-BSA antibodies

| Group | ELISA Units | SEM |
|---|---|---|
| BSA in saline | 0 | |
| BSA + 50 mM Liposomes | 0 | |
| CT + BSA in saline | 8,198 | (5,533-11,932) |
| CT + BSA + 50 mM | 3,244 | (128-3,242) |

Example 3

BALB/c mice at 6 to 8 weeks of age were immunized transcutaneously as described above for "Immunization Procedure", in groups of five mice. The mice were immunized at 0 and 3 weeks using 100 $\mu$l of immunization solution prepared as follows: LT was mixed in saline to make an immunizing solution containing 100 $\mu$g of LT per 100 $\mu$l of saline for the group receiving LT alone. Where liposomes were used the liposomes prepared as described above for "Liposome Preparation", and were first mixed with saline to form the liposomes. The pre-formed liposomes were then diluted in LT in saline to yield an immunizing solution containing liposomes at 50 mM phospholipid with 100 $\mu$g of LT per 100 $\mu$l of immunizing solution. Solutions were vortexed for 10 seconds prior to immunization.
The anti-LT antibodies were determined using ELISA as described above for "ELISA IgG(H+L)" 3 weeks after the second immunization. The results are shown in Table 3. LT was clearly immunogenic both with and without liposomes, and no significant difference between the groups could be detected. LT and CT are members of the family of bacterial ADP-ribosylating exotoxins (bAREs). They are organized as A:B proenzymes with the ADP-ribosyltransferase activity contained in the A subunit and the target cell binding a function of the B subunit. LT is 80% homologous with CT at the amino acid level and has a similar non-covalently bound subunit organization, stoichiometry (A:B5), the same binding target, ganglioside GM1, and is similar in size (MW ~80,000). The similarities of LT and CT appear to influence their immunogenicity by the transcutaneous route as reflected by the similar magnitude of the antibody response to both CT and LT (Tables 1 and 3).

Table 3. Anti-LT antibodies

| Group | ELISA Units | SEM |
| --- | --- | --- |
| LT in saline | 23,461 | (20,262-27,167) |
| LT + 50 mM Liposomes | 27,247 | (19,430-38,211) |

Example 4

C57B1/6 mice at 6 to 8 weeks of age were immunized transcutaneously as described above for "Immunization Procedure", in groups of five mice. The mice were immunized once using 100 $\mu$l of immunization solution prepared as follows: LT was mixed in saline to make an immunizing solution containing 100 $\mu$g of LT per 100 $\mu$l of saline. The solution was vortexed for 10 seconds prior to immunization.

The anti-LT antibodies were determined using ELISA as described above for "ELISA IgG (H+L)" 3 weeks after the single immunization. The results are shown in Table 4. LT was clearly immunogenic with a single immunization and antibodies were produced by 3 weeks. Rapid enhancement of antibody titers and responses to single immunization would be a useful aspect of the transcutaneous immunization method. It is conceivable that a rapid single immunization would be useful in epidemics, for travelers, and where access to medical care is poor.

Table 4. Anti-LT antibodies

| Mouse Number | ELISA Units |
| --- | --- |
| 5141 | 6,582 |
| 5142 | 198 |
| 5143 | 229 |
| 5144 | 6, 115 |
| 5145 | 17,542 |
| Geo Mean | 2,000 |

Example 5

C57B1/6 mice at 8 to 12 weeks of age were immunized transcutaneously as described above for "Immunization Procedure", in groups of five mice. The mice were immunized once using 100 $\mu$l of immunization solution prepared as follows: CT was mixed in saline to make an immunizing solution containing 100 $\mu$g of CT per 100 $\mu$l of saline. The solution was vortexed for 10 seconds prior to immunization.

The anti-CT antibodies were determined using ELISA as described above for "ELISA IgG (H+L)" 3 weeks after the single immunization. The results are shown in Table 5. CT was highly immunogenic with a single immunization. Rapid enhancement of antibody titers and responses to single immunization may be a useful aspect of the transcutaneous immunization method. It is conceivable that a rapid single immunization would be useful in epidemics, for travelers, and where access to medical care is poor.

Table 5. Anti-CT antibodies

| Mouse Number | ELISA Units |
| --- | --- |
| 2932 | 18,310 |
| 2933 | 30,878 |
| 2934 | 48,691 |
| 2935 | 7,824 |
| Geo Mean | 21,543 |

Example 6

BALB/c mice at 6 to 8 weeks of age were immunized transcutaneously as described above for "Immunization Procedure", in groups of five mice. The mice were immunized at 0 and 3 weeks using 100 $\mu$l of immunization solution prepared as follows: ETA was mixed in saline to make an immunizing solution containing 100 $\mu$g of ETA per 100 $\mu$l of saline for the group receiving ETA alone. Where liposomes were used, the liposomes were prepared as described above for "Liposome Preparation", and were first mixed with saline to form the liposomes. The pre-formed liposomes were then diluted with ETA in saline to yield an immunizing solution containing liposomes at 50 mM phospholipid with 100 μg of ETA per 100 μl of immunizing solution. Solutions were vortexed for 10 seconds prior to immunization.

The antibodies were determined using ELISA as described above for "ELISA IgG(H+L)" on sera 3 weeks after the second immunization. The results are shown in Table 6. ETA was clearly immunogenic both with and without liposomes, and no significant difference between the groups could be detected. ETA differs from CT and LT in that ETA is a single 613 amino acid peptide with A and B domains on the same peptide and binds to an entirely different receptor, the α2-macroglobulin receptor/low density lipoprotein receptor-related protein (Kounnas et al, 1992). Despite the dissimilarities between ETA and CT in size, structure, and binding target, ETA also induced a transcutaneous antibody response.

Table 6. Anti-ETA antibodies

| Group | ELISA Units | SEM |
|---|---|---|
| ETA in saline | 3,756 | (1,926-7,326) |
| ETA + 50 mM Liposomes | 857 | (588-1,251) |

Example 7

BALB/c mice at 6 to 8 weeks of age were immunized transcutaneously as described above for "Immunization Procedure", in groups of five mice. The mice were immunized using 100 μl of immunization solution which was prepared as follows: CT was mixed in saline to make 100 μg of CT per 100 μl of immunizing solution, LT was mixed in saline to make 100 μg of LT per 100 μl of immunizing solution, ETA was mixed in saline to make 100 μg of ETA per 100 μl of immunizing solution, and CT and BSA were mixed in saline to make 100 μg of CT per 100 μl of immunizing solution and 200 μg of BSA per 100 μl of immunizing solution. Solutions were vortexed for 10 seconds prior to immunization.

The mice were immunized transcutaneously at 0 and 3 weeks and the antibody levels were determined using ELISA as described above for "ELISA IgG Subclass", three weeks after the boosting immunization and compared against the pre-immune sera. The IgG subclass response to CT, BSA and LT had similar levels of IgG1 and IgG2a reflecting activation of T help from both Th1 and Th2 lymphocytes (Seder and Paul, 1994), whereas the IgG subclass response to ETA consisted of almost exclusively IgG1 and IgG3, consistent with a Th2-like response (Table 7). Thus, it appears that all IgG subclasses can be produced using transcutaneous immunization.

Table 7. IgG subclasses of induced antibodies

| Imm. Antigen | Antibody Specificity | IgG1 (μg/μl) | IgG2a (μg/μl) | IgG2b (μg/μl) | IgG3 (μg/μl) |
|---|---|---|---|---|---|
| CT | CT | 134 | 25 | 27 | 0 |
| CT+BSA | BSA | 108 | 17 | 12 | 5 |
| LT | LT | 155 | 28 | 10 | 8 |
| ETA | ETA | 50 | 0 | 1 | 10 |

Example 8

BALB/c mice at 6 to 8 weeks of age were immunized transcutaneously as described above for "Immunization Procedure", in groups of five mice. The mice were immunized using 100 μl of immunization solution which was prepared as follows: LT was mixed in saline to make an immunizing solution containing 100 μg of LT per 100 μl of saline for the group receiving LT alone, CT was mixed in saline to make an immunizing solution containing 100 μg of CT per 100 μl of saline for the group receiving CT alone, ETA was mixed in saline to make an immunizing solution containing 100 μg of ETA per 100 μl of saline for the group receiving ETA alone, and BSA and CT were mixed in saline to make an immunizing solution containing 100 μg of BSA and 100 μg of CT per 100 μl of saline for the group receiving BSA and CT.

The mice were immunized transcutaneously at 0 and 3 weeks and the antibody levels were determined using ELISA as described above for "ELISA IgE", one week after the boosting immunization and compared against the pre-immune sera. As shown in Table 8, no IgE antibodies were found although the sensitivity of detection was 0.003 μg/ml. IgG antibodies were determined in the same mice using "ELISA IgG(H+L)" on sera 3 weeks after the second immunization. The IgG antibody response to LT, ETA, CT and BSA are shown to indicate that the animals were successfully immunized and responded with high titers of antibodies to the respective antigens.

Table 8. IgE antibodies to LT, ETA, CT and BSA

| Group | Antibody Specificity | IgE ($\mu$g/ml) | IgG (ELISA Units) |
|---|---|---|---|
| LT | Anti-LT | 0 | 23,461 |
| ETA | Anti-ETA | 0 | 3,756 |
| CT | Anti-CT | 0 | 39,828 |
| CT + BSA | Anti-BSA | 0 | 8,198 |

Example 9

BALB/c mice at 6 to 8 weeks of age immunized transcutaneously as described above for "Immunization Procedure", in groups of five mice. The mice were immunized at 0 and 3 weeks using 100 ml of immunization solution which was prepared as follows: CT was mixed in saline to make an immunizing solution containing 100 mg of CT per 100 ml of immunizing solution. The immunization solution was vortexed for 10 seconds prior to immunization.

The mice were immunized transcutaneously at 0 and 3 weeks and the antibody levels were determined using ELISA as described above for "ELISA IgG(H+L)" and "ELISA IgG($\gamma$)". Determinations were done at 1 and 4 weeks after the initial immunization, and compared against the pre-immune sera. As shown in Table 9, high levels of anti-CT IgG($\gamma$) antibodies were induced by CT in saline. Small amounts of IgM could be detected by using IgM($\mu$) specific secondary antibody. By 4 weeks, the antibody response was primarily IgG. Data are reported in ELISA units.

Table 9. IgG($\gamma$) and IgM($\mu$)

| Imm. Group | Week | IgG ($\gamma$) | IgM ($\mu$) |
|---|---|---|---|
| CT | 1 | 72 | 168 |
| CT | 4 | 21,336 | 38 |
| L()+CT | 1 | 33 | 38 |
| L()+CT | 4 | 22,239 | 70 |

Example 10

BALB/c mice at 6 to 8 weeks of age were immunized transcutaneously as described above for "Immunization Procedure", in groups of five mice. The mice were immunized once using 100 $\mu$l of immunization solution prepared as follows: CT was mixed in saline to make an immunizing solution containing 100 $\mu$g of CT per 100 $\mu$l of saline. The solution was vortexed for 10 seconds prior to immunization. The mice were immunized transcutaneously at 0 and 3 weeks. Antibody levels were determined using ELISA as described above for "ELISA IgG (H+L)" 5 weeks after the boosting immunization, and compared against pre-immune sera. As shown in Table 10, serum anti-CT IgA antibodies were detected.

Table 10. Anti-CT IgA antibodies

| Mouse Number | IgA (ng/ml) |
|---|---|
| 1501 | 232 |
| 1502 | 22 |
| 1503 | 41 |
| 1504 | 16 |
| 1505 | 17 |

Example 11

BALB/c mice at 6 to 8 weeks of age were immunized transcutaneously as described above for "Immunization Procedure", in groups of five mice. The mice were immunized using 100 $\mu$l of immunization solution which was prepared as follows: CT was mixed in saline to make an immunizing solution containing 100 $\mu$g of CT per 100 $\mu$l of immunizing solution. The immunization solution was vortexed for 10 seconds prior to immunization.

The mice were immunized with 100 $\mu$l of immunizing solution transcutaneously at 0 and 3 weeks and the antibody levels were determined using ELISA as described above for "ELISA IgG(H+L)" and "ELISA IgG($\gamma$)". Antibody

determinations were done at 8 weeks after the initial immunization and compared against the pre-immune sera. As shown in Table 11, high levels of serum anti-CT antibodies were induced by CT in saline. Lung wash IgG could be detected by ELISA using IgG(H+L) or IgG($\gamma$) specific antibody. The antibody found on the lung mucosal surface is diluted by the lavage method used to collect mucosal antibody and, thus, the exact amounts of antibody detected are not as significant as the mere presence of detectable antibody.

Lung washes were obtained after sacrificing the mouse. The trachea and lungs were exposed by gentle dissection and trachea was transected above the bifurcation. A 22 gauge polypropylene tube was inserted and tied off on the trachea to form a tight seal at the edges. Half a milliliter of PBS was infused using a 1 ml syringe attached to the tubing and the lungs were gently inflated with the fluid. The fluid was withdrawn and reinfused for a total of 3 rounds of lavage. The lung wash was then frozen at - 20°C.

Table 11 shows the IgG(H+L) and IgG($\gamma$) antibody response to cholera toxin in the sera and lung washes at 8 weeks. Data are expressed in ELISA units. Antibodies were clearly detectable for all mice in the lung washes. The presence of antibodies in the mucosa may be important for protection against mucosally active diseases.

Table 11. Mucosal Antibody to CT

| Animal# | Imm. Group | IgG(H+L) | IgG($\gamma$) | Source |
|---|---|---|---|---|
| 1501 | CT | 133 | 34 | Lungs |
| 1502 | CT | 75 | 12 | Lungs |
| 1503 | CT | 162 | 28 | Lungs |
| 1504 | CT | 144 | 18 | Lungs |
| 1505 | CT | 392 | 56 | Lungs |
| | Geo Mean | 156 | 26 | |
| 1501 | CT | 34,131 | 13,760 | Sera |
| 1502 | CT | 11,131 | 2,928 | Sera |
| 1503 | CT | 21,898 | 10,301 | Sera |
| 1504 | CT | 22,025 | 8,876 | Sera |
| 1505 | CT | 34,284 | 10,966 | Sera |
| | Geo Mean | 23,128 | 8,270 | |

Example 12

BALB/c mice were immunized transcutaneously at 0 and 3 weeks as described above for "Immunization Procedure", in groups of four mice. Liposomes were prepared as described above for "Liposome Preparation", and were first mixed with saline to form the liposomes. The pre-formed liposomes were then diluted with either CT, CTA or CTB in saline to yield an immunizing solution containing liposomes at 50 mM phospholipid with 50 $\mu$g of antigen (CT, CTA or CTB) per 100 $\mu$l of immunizing solution. Solutions were vortexed for 10 seconds prior to immunization.

The antibodies were determined using ELISA as described above for "ELISA IgG(H+L)", one week after the boosting immunization and compared against the pre-immune sera. The results are shown in Table 12. CT and CTB were clearly immunogenic whereas CTA was not. Thus, the B subunit of CT is necessary and sufficient to induce a strong antibody response.

Table 12. Antibodies to CT, CTA and CTB

| Group | Anti-CT | Anti-CTA | Anti-CTB |
|---|---|---|---|
| CT + 50 mM Liposomes | 12,636 | 136 | 7,480 |
| CTB + 50 mM Liposomes | 757 | 20 | 1,986 |
| CTA + 50 mM Liposomes | 0 | 0 | 0 |

Example 13

BALB/c mice were immunized transcutaneously as described above for "Immunization Procedure", in groups of five mice. Mice were immunized at 0 and 3 weeks with 100 µg of diphtheria toxoid and 10 µg of pertussis toxin per 100 µl of saline solution. Solutions were vortexed for 10 seconds prior to immunization.

The antibodies were quantitated using ELISA as described for "ELISA IgG(H+L)". Anti-diphtheria toxoid antibodies were detected only in animals immunized with both pertussis toxin and diphtheria toxoid. The highest responder had anti-diphtheria toxoid antibody ELISA units of 1,038. Thus, a small amount of pertussis toxin acts as an adjuvant for diphtheria toxoid antigen. The toxoid alone did not induce an immune response suggesting that the toxoiding process has affected the portion of the molecule responsible for the adjuvant effects found in the ADP-ribosylating exotoxin.

Table 13. Antibody to Diphtheria

| Mouse Number | Immunizing Antigen | IgG ELISA Units |
| --- | --- | --- |
| 4731 | DT + PT | 1,039 |
| 4732 | DT + PT | 1 |
| 4733 | DT + PT | 28 |
| 4734 | DT + PT | 15 |
| 4735 | DT + PT | 20 |
| 4621 | DT | 0 |
| 4622 | DT | 0 |
| 4623 | DT | 0 |
| 4624 | DT | 0 |
| 4625 | DT | 0 |

Example 14

BALB/c mice were immunized transcutaneously as described above for "Immunization Procedure", in groups of five mice. Mice were immunized once at 0, 8 and 20 weeks with 50 µg of pertussis toxin (List, catalog # 181, lot #181-20a) per 100 µl of saline solution.

The antibodies were quantitated using ELISA as described for "ELISA IgG(H+L)". Anti-pertussis toxin antibodies were detected one week after the last boost in animals immunized with pertussis. All five animals had elevated levels of anti-petussis toxin antibody after the last immunization. Thus, pertussis toxin acts as an adjuvant for itself and induces PT-specific PT-specific IgG antibodies. The adjuvant effect of PT may be useful in combination vaccines such as Diphtheria/Pertussis/Tetanus/Hib in enhancing the antibody response to coadministered antigens as well as to PT itself.

Table 14. Antibody response to Pertussis toxin

| Mouse Number | Antigen | 2 weeks | 21 weeks |
| --- | --- | --- | --- |
| 5156 | PT | 14 | 256 |
| 5157 | PT | 22 | 330 |
| 5158 | PT | 17 | 303 |
| 5159 | PT | 33 | 237 |
| 5160 | PT | 75 | 418 |

Example 15

BALB/c mice were immunized transcutaneously as described above for "Immunization Procedure", in groups of five mice. Mice were immunized once at 0 weeks with 50 µg of tetanus toxoid and 100 µg of cholera toxin per 100 µl of saline solution.

The antibodies were quantitated using ELISA as described for "ELISA IgG(H+L)". Anti-tetanus toxoid antibodies were detected at 8 weeks in animal 5173 at 443 ELISA units.

Example 16

The possibility that oral immunization occurred through grooming after epicutaneous application and subsequent washing of the site of application was evaluated using $^{125}$I-labeled CT to track the fate of the antigen/adjuvant. Mice were anesthetized, transcutaneously immunized as described above for "Immunization Procedure" with 100 $\mu$g of $^{125}$I-labeled CT (150,000 cpm/$\mu$g CT). Control mice remained anesthetized for 6 hours to exclude grooming, and experimental mice were anesthetized for one hour and then allowed to groom after washing. Mice were sacrificed at 6 hours and organs weighed and counted for $^{125}$I on a Packard gamma counter. A total of 2-3 $\mu$g of CT was detected on the shaved skin at the site of immunization (14,600 cpm/$\mu$g tissue) while a maximum of 0.5 $\mu$g of CT was detected in the stomach (661 cpm/$\mu$g tissue) and intestine (9 cpm/$\mu$g tissue).

Oral immunization (n=5) with 10 $\mu$g if CT in saline at 0 and 3 weeks (without NaHCO$_3$) induced a 6 week mean IgG antibody response of < 1,000 ELISA units whereas transcutaneous immunization with 100 $\mu$g of CT, shown above to result in less than 5 $\mu$g of CT retained in the skin after washing, resulted in an anti-CT response of 42,178 ELISA units at 6 weeks. Induction of an immune response to orally fed CT requires the addition of NaHCO$_3$ to the immunizing solution (Piece, 1978; Lycke and Holmgren, 1986). Thus, oral immunization does not significantly contribute to the antibodies detected when CT is applied epicutaneously to the skin.

Example 17

In vivo evidence of Langerhans cell activation was obtained using cholera toxin (CT) in saline applied epicutaneously to the skin, specifically the ears of the mouse, where large populations of Langerhans cells can be readily visualized (Enk et al, 1993; Bacci et al, 1997), and staining for major histocompatibility complex (MHC) class II molecules which is upregulated in activated Langerhans cells (Shimada et al, 1987).

BALB/c mouse ears were coated on the dorsal side with either 100 $\mu$g of CT in saline, 100 $\mu$g of CTB in saline, saline alone, or an intradermal injection of the positive controls 100 pg LPS or 10 $\mu$g TNF$\alpha$, for one hour while the mouse was anesthetized. The ears were then thoroughly washed and, after 24 hours, the ears were removed and epidermal sheets were harvested and stained for MHC class II expression as described by Caughman et al (1986). Epidermal sheets were stained with MKD6 (anti-I-A$^d$) or negative control Y3P (anti-I-A$^k$), and goat anti-mouse FITC F(ab)$_2$ was used as a second step reagent. Mice transcutaneously immunized on the ear (as described above without shaving) had previously been found to have anti-CT antibodies of 7,000 ELISA units three weeks after a single immunization.

Enhanced expression of MHC class II molecules as detected by staining intensity, the reduced number of Langerhans cells (especially with cholera toxin), and changes in Langerhans cell morphology were found in the epidermal sheets of the mice immunized with CT and CTB comparable to controls (Fig. 1), suggesting that the Langerhans cells were activated by the epicutaneously applied cholera toxin (Aiba and Katz, 1990; Enk et al, 1993).

Example 18

Langerhans cells represent the epidermal contingent of a family of potent accessory cells termed 'dendritic cells'. Langerhans cells (and perhaps related cells in the dermis) are thought to be required for immune responses directed against foreign antigens that are encountered in skin. The 'life cycle' of the Langerhans cell is characterized by at least two distinct stages. Langerhans cells in epidermis (the 'sentinels') can ingest particulates and process antigens efficiently, but are weak stimulators of unprimed T cells. In contrast, Langerhans cells that have been induced to migrate to lymph nodes after contact with antigen in epidermis (the 'messengers') are poorly phagocytic and have limited antigen-processing capabilities, but are potent stimulators of naive T cells. If Langerhans cells are to fulfill both their 'sentinel' and 'messenger' roles, they must be able to persist in epidermis, and also be able to exit epidermis in a controlled fashion after exposure to antigen. Thus, regulation of Langerhans cell-keratinocyte adhesion represents a key control point in Langerhans cell trafficking and function.

Langerhans cells express E-cadherin (Blauvelt et al, 1995), a homophilic adhesion molecule that is prominently represented in epithelia. Keratinocytes also express this adhesion molecule, and E-cadherin clearly mediates adhesion of murine Langerhans cells to keratinocytes in vitro. It is known that E-cadherin is involved in the localization of Langerhans cells in epidermis. See Stingl et al (1989) for a review of the characterization and properties of Langerhans cells and keratinocytes.

The migration of epidermal Langerhans cells (LC) and their transport of antigen from the skin to draining lymph nodes are known to be important in the induction of cutaneous immune responses, such as contact sensitization. While in transit to the lymph nodes, Langerhans cells are subject to a number of phenotypic changes required for their movement from the skin and acquisition of the capacity for antigen presentation. In addition to the upregulation of MHC class II molecules, are alterations in the expression of adhesion molecules that regulate interactions with the surrounding tissue matrix and with T lymphocytes. The migration of the Langerhan cell is known to be associated with a marked reduction in the expression of E-cadherin (Schwarzenberger and Udey, 1996, and a parallel upregulation of ICAM-1 (Udey, 1997).

Transcutaneous immunization with bacterial ADP ribosylating exotoxins (bARE's) target the Langerhans cells in the epidermis. The bAREs activate the Langerhans cell, transforming it from its sentinel role to its messenger role. Ingested antigen is then taken to the lymph node where it is presented to B and T cells (Streilein and Grammer, 1989;

Kripke et al, 1990; Tew et al, 1997). In the process, the epidermal Langerhans cell matures into an antigen-presenting dendritic cell in the lymph node (Schuler and Steinman, 1985); lymphocytes entering a lymph node segregate into B-cell follicles and T-cell regions. The activation of the Langerhans cell to become a migratory Langerhans cell is known to be associated with not only a marked increase in MHC class II molecules, but also marked reduction in the expression of E-cadherin, and upregulation of ICAM-1.

We envision that cholera toxin (CT) and its B subunit (CTB) upregulate the expression of ICAM-1 and downregulate the expression of E-cadherin on Langerhans cells as well as upregulate the expression of MHC class II molecules on the Langerhans cell. CT or CTB acts as an adjuvant by freeing the sentinel Langerhans cell to present antigens such as BSA or diphtheria toxoid phagocytosed by the Langerhans cell at the same location and time as the encounter with the CT or CTB when they are acting as adjuvant. The activation of a Langerhans cells to upregulate the expression of ICAM-1 and dowregulate the expression of E-cadherin may be mediated by cytokine release including TNFα and IL-1β from the epidermal cells or the Langerhans cells themselves.

This method of adjuvancy for transcutaneous immunization is envisioned to work for any compound that activates the Langerhans cell. Activation could occur in such manner as to downregulate the E-cadherin and upregulate ICAM-1. Langerhans cells would then carry antigens made of mixtures of such Langerhans cell-activating compounds and antigens (such as diphtheria toxoid or BSA) to the lymph nodes where the antigens are presented to T cells and evoke an immune response. Thus, the activating substance such as a bARE can be used as an adjuvant for an other wise transcutaneously non-immunogenic antigen such as Diphtheria toxoid by activating the Langerhans cell to phagocytose the antigen such as diphtheria toxoid, migrate to the lymph node, mature into a dendritic cell, and present the antigen to T cells.

The T-cell helper response to antigens used in transcutaneous immunization may be influenced by the application of cytokines and/or chemokines. For example, interleukin-10 (IL-10) may skew the antibody response towards a Th2 IgG1/IgE response whereas anti-IL-10 may enhance the production of IgG2a (Bellinghausen et al, 1996).

Example 19

Sequestrin is a molecule expressed on the surface of malaria-infected erythrocytes which functions to anchor the malaria parasitized red blood cell to vascular endothelium. This is essential for parasite survival and contributes directly to the pathogenesis of *P. falciparum* malaria in children dying of cerebral malaria. In cerebral malaria, the brain capillaries become plugged with vast numbers of parasitized red blood cells due to the specific interaction of the sequestrin molecule with the host endothelial receptor CD36. Ockenhouse et al identified both the host receptor CD36 and parasite molecule (sequestrin) which mediates this receptor-ligand interaction. Ockenhouse et al have cloned and expressed as *E. coli*-produced recombinant protein the domain of the sequestrin molecule which interacts with the CD36 receptor. A truncated 79 amino acid sequestrin product was used in the example below.

Active immunization with recombinant sequestrin or DNA encoding the gene for sequestrin should elicit antibodies which block the adhesion of malaria parasitized erythrocytes to host endothelial CD36 and thereby prevent completion of parasite life cycle leading to parasite death due to its inability to bind to endothelium. The strategy is to develop a method of immunization which elicits high titer blocking antibodies. One such method is the deliver the vaccine transcutaneously. Measurement of both total antibody titers as well as blocking activity and opsonization forms the basis for this approach with transcutaneous immunization. The recombinant sequestrin protein used in the present experiments is 79 amino acids long (-18 kDa) and comprises the CD36-binding domain of the molecule. We have also constructed a naked DNA construct comprised of this domain and have elicited antibodies using epidermal gene gun delivery.

BALB/c mice (n=3) were immunized transcutaneously as described above for "Immunization Procedure". The mice were immunized at 0 and 8 weeks using 120 μl of immunization solution prepared as follows: a plasmid encoded for *P. falciparum* sequestrin was mixed in saline to make an immunizing solution containing 80 μg of plasmid, 80 μg of CT (List Biologicals) per 100 μl of saline. One hundred-twenty μl was applied to the untagged ear after gently cleansing the ear with an alcohol swab (Triad Alcohol pad, 70% isopropyl alcohol). The immunizing solution was not removed by washing.

The antibodies to sequestrin were determined using ELISA as described above for "ELISA IgG(H+L)" on sera collected from the tail vein at weeks 3, 4, 7 and 9 after the primary immunization. The results are shown in Table 15.

Sequestrin DNA with CT induced a detectable antibody response to the expressed protein after the second boosting immunization. For immunization to occur, the protein needs to be expressed and processed by the immune system. Thus, CT acted as an adjuvant for the immune response to sequestrin protein expressed by the plasmid encoding for sequestrin.

DNA vaccines have been shown to elicit neutralizing antibodies and CTLs in non-human primates to diseases such as malaria (Gramzinski, Vaccine, 15:913-915, 1997) and HIV (Shriver et al, Vaccine 15:884-887, 1997) and have demonstrated protection to varying degrees in several models (McClements et al, Vaccine, 15:857-60, 1997). DNA immunization through the skin could be expected to elicit responses similar to that of the gene gun which targets the skin immune system (Prayaga et al, Vaccine, 15:1349-1352, 1997).

Table 15. Serum antibody against sequestrin (Seq) protein in animals immunized with Seq DNA and Cholera toxin (CT)

| Animal # | Imm. Group | IgG (H+L) ELISA Units | | | |
|---|---|---|---|---|---|
| | | week 3 | week 4 | week 7 | week 9 |
| 8966 | Seq DNA/CT | 58 | 80 | 33 | - |
| 8967 | Seq DNA/CT | 76 | 81 | 41 | 146 |
| 8968 | Seq DNA/CT | 54 | 33 | 26 | - |
| | Geo Mean | 62 | 60 | 33 | |
| pooled prebleed | - | 40 | | | |

Example 20

BALB/c mice were immunized transcutaneously as described above for "Immunization Procedure", in groups of five mice, using sequestrin. The mice were immunized at 0, 2 and 8 weeks using 100 $\mu$l of immunization solution prepared as follows: at 0 weeks the mice were immunized with 59 $\mu$g of CT and 192 $\mu$g of sequestrin in 410 $\mu$l for the group receiving sequestrin and CT, 192 $\mu$g in 410 $\mu$l for sequestrin alone, and 120 $\mu$g of CTB and 250 $\mu$g of sequestrin in 520 $\mu$l for the group receiving sequestrin and CTB. Two weeks later the mice were boosted with 345 $\mu$l of saline containing either 163 $\mu$g sequestrin for the sequestrin alone group, 345 $\mu$l of saline containing 163 $\mu$g sequestrin with 60 $\mu$g of CT for the CT plus sequestrin group, 345 $\mu$l of saline containing 163 $\mu$g sequestrin and 120 $\mu$g of CTB for the sequestrin plus CTB group. In the second boost the mice were given 120 $\mu$g of sequestrin for the sequestrin alone group, 120 $\mu$g of sequestrin and 120 $\mu$g of CT for the CT plus sequestrin group and 120 $\mu$g of sequestrin and 120 $\mu$g of CTB for the sequestrin plus CTB group.

The antibodies were determined using ELISA as described above for "ELISA IgG(H+L)" on sera 3, 5, 7, 9, 10, 11 and 15 weeks after the first immunization. The results are shown in Table 16. Sequestrin alone induced a small but detecable antibody response. However, the addition of CT stimulated a far stronger immune response to sequestrin and CTB induced an immune response that was superior to sequestrin alone. CT and CTB acted as adjuvants for the immune response to sequestrin, a recombinant protein.

Table 16. Seq, Seq + Cholera toxin (CT), or Seq + Cholera toxin B (CTB)

| Animal# | Immunization Group | Detecting Antigen | IgG (H+L) ELISA Units | | | | | | | |
|---|---|---|---|---|---|---|---|---|---|---|
| | | | prebleed | week 3 | week 5 | week 7 | week 8 | week 9 | week 11 | week 15 |
| 2861 | Seq | Seq | | 7 | 7 | 20 | 32 | 709 | 431 | 408 |
| 2862 | Seq | Seq | | 8 | 5 | 14 | 136 | 33 | 4 | 6 |
| 2863 | Seq | Seq | | 28 | 63 | 38 | 393 | 467 | 348 | 459 |
| 2864 | Seq | Seq | | 5 | 9 | 26 | 102 | 32 | 13 | 11 |
| 2865 | Seq | Seq | | 9 | 19 | 76 | 111 | 100 | 53 | 98 |
| | | Geo Mean | | 9 | 13 | 29 | 114 | 129 | 54 | 65 |
| 2866 | Seq/CT | Seq | | 923 | 1145 | 125 | 639 | 43679 | 28963 | 42981 |
| 2867 | Seq/CT | Seq | | 73 | 84 | 154 | ND | 9428 | 20653 | 27403 |
| 2868 | Seq/CT | Seq | | 805 | 370 | 1447 | 1105 | ND | 13169 | 7677 |
| 2869 | Seq/CT | Seq | | 175 | 760 | 1317 | 768 | 113792 | 118989 | 270040 |
| 2870 | Seq/CT | Seq | | 153 | 158 | 535 | 241 | 3245 | ND | 4277 |
| | | Geo Mean | | 271 | 336 | 456 | 601 | 19747 | 31115 | 25279 |
| 2871 | Seq/CTB | Seq | | 8 | 3 | 87 | 40 | 22 | 29 | 192 |
| 2872 | Seq/CTB | Seq | | 4 | 6 | 24 | 22 | 35 | 24 | 34 |
| 2873 | Seq/CTB | Seq | | 107 | 138 | 128 | 51 | 2079 | 2283 | 2296 |
| 2874 | Seq/CTB | Seq | | 6 | 7 | 22 | 18 | 41 | 40 | 457 |
| 2875 | Seq/CTB | Seq | | 515 | 504 | 1910 | 1744 | ND | 7148 | 5563 |
| | | Geo Mean | | 25 | 25 | 102 | 68 | 91 | 214 | 520 |
| pool | | | 5 | | | | | | | |

Example 21

BALB/c mice were immunized transcutaneously as described above for "Immunization Procedure", in groups of five mice. The mice were immunized at 0 weeks using 100 µl of immunization solution prepared as follows: FLUSHIELD (Wyeth-Ayerst, purified subvirion, 1997-98 formula, lot #U0980-35-1) was lyophilized and was mixed in saline to make an immunizing solution containing 90 µg of FLUSHIELD subvirion per 100 µl of saline for the group receiving influenza alone; influenza and CT were mixed in saline to make an immunizing solution containing 90 µg of FLUSHIELD antigens and 100 µg of CT per 100 µl of saline for the group receiving influenza and CT.

The antibodies were determined using ELISA as described above for "ELISA IgG(H+L)" on sera 3 weeks after the first immunization. The results are shown in Table 17. Influenza alone did not induce an antibody response. However, the addition of CT stimulated a far stronger immune response which was superior to that observed influenza alone. Thus CT acted as an adjuvant for the immune response to FLUSHIELD, subvirion influenza vaccine, a mixture of virally derived antigens.

Table 17. Serum antibody against Influenza (Inf) types A and B in animals immunized with Inf alone or Inf + Cholera toxin (CT)

| | | IgG (H+L) |
| | | ELISA Units |
| Animal # | Imm. Group | week 3 |
|---|---|---|
| 8601 | CT/Inf | 144 |
| 8602 | CT/Inf | 14 |
| 8603 | CT/Inf | 1325 |
| 8604 | CT/Inf | 36 |
| 8605 | CT/Inf | 29 |
| | Geo Mean | 77 |
| 8606 | Inf | 17 |
| 8607 | Inf | 16 |
| 8608 | Inf | 20 |
| 8609 | Inf | 23 |
| 8610 | Inf | 23 |
| | Geo Mean | 20 |

Example 22

LT is in the family of ADP-ribosylating exotoxins and is similar to CT in molecular weight, binds to ganglioside GM1, is 80% homologous with CT and has a similar A:B5 stoichiometry. Thus, LT was also used as an adjuvant for DT in transcutaneous immunization. BALB/c mice (n=5) were immunized as described above at 0, 8 and 18 weeks with a saline solution containing 100 µg of LT (Sigma, catalog #E-8015, lot 17hH12000 and 100 µg CT (List Biologicals, catalog #101b) in 100 µl of saline. LT induced a modest response to DT as shown in Table 18.

ETA (List Biologicals, lot #ETA 25A) is in the family of ADP-ribosylating exotoxins, but is a single polypeptide that binds to a different receptor. One hundred µg of ETA was delivered in 100 µl of a saline solution containing 100 µg of CT to BALB/c mice on the back as previously described at 0, 8 and 18 weeks. ETA boosted the response to DT at 20 weeks. Thus, other ADP-ribosylating exotoxins were able to act as adjuvants for coadministered proteins (Table 18).

Table 18. Kinetics of Diphtheria toxoid (DT) antibody titers in animals immunized with *Pseudomonas aeruginosa* exotoxin A (ETA) and DT or *E. coli* heat labile enterotoxin (LT) and DT

| | Immunization | Detecting | IgG (H+L) | ELISA Units |
| Animal # | Group | Antigen | prebleed | week 20 |
|---|---|---|---|---|
| 5146 | ETA/DT | DT | | 31718 |

(continued)

|  | Immunization | Detecting | IgG (H+L) | ELISA Units |
|---|---|---|---|---|
| Animal # | Group | Antigen | prebleed | week 20 |
| 5147 | ETA/DT | DT |  | 48815 |
| 5148 | ETA/DT | DT |  | 135 |
| 5149 | ETA/DT | DT |  | 34 |
| 5150 | ETA/DT | DT |  | 258 |
|  |  | Geo Mean |  | 1129 |
| 5136 | LT/DT | DT |  | 519 |
| 5137 | LT/DT | DT |  | 539 |
| 5138 | LT/DT | DT |  | 38 |
| 5139 | LT/DT | DT |  | 531 |
| 5140 | LT/DT | DT |  | 901 |
|  |  | Geo Mean |  | 348 |
| pool |  |  | 3 |  |

Example 23

BALB/c mice were immunized transcutaneously as described above for "Immunization Procedure" in groups of five mice. Mice were immunized at 0 weeks, 8 weeks and 18 weeks with 100 μl saline containing 100 μg Cholera toxin (List Biologicals, catalog #101B, lot #10149CB), 50 μg Tetanus toxoid (List Biologicals, catalog # 191B, lots #1913a and 1915b) and 83 μg Diphtheria toxoid (List Biologicals, catalog #151, lot #15151).

The antibodies against CT, DT, and TT were quantitated using ELISA as described for "ELISA IgG (H+L)". Anti-CT, DT, or TT antibodies were detected at 23 weeks following the primary immunization. Anti-Diphtheria toxoid and Cholera toxin antibodies were elevated in all immunized mice. The highest responder had anti-tetanus toxoid antibody ELISA units of 342, approximately 80 times the level of antibody detected in sera of unimmunized animals. Thus, a combination of unrelated antigens (CT/TT/DT) can be used to immunize against the individual antigens. This demonstrates that Cholera toxin can be used as an adjuvant for multivalent vaccines.

Table 19. Serum antibody in animals immunized simultaneously with Cholera toxin, Tetanus toxoid, and Diphtheria toxoid

|  |  |  | IgG (H+L) ELISA Units | |
|---|---|---|---|---|
| Animal # | Imm. Group | detecting antigen | prebleed | 23 weeks |
| 5176 | CT/TT/DT | CT |  | 7636 |
| 5177 | CT/TT/DT | CT |  | 73105 |
| 5179 | CT/TT/DT | CT |  | 126259 |
| 5216 | CT/TT/DT | CT |  | 562251 |
| 5219 | CT/TT/DT | CT |  | 66266 |
| pool |  |  | ≤3 |  |
|  | Geo Mean |  |  | 76535 |
| 5176 | CT/TT/DT | DT |  | 64707 |
| 5177 | CT/TT/DT | DT |  | 17941 |
| 5179 | CT/TT/DT | DT |  | 114503 |
| 5216 | CT/TT/DT | DT |  | 290964 |
| 5219 | CT/TT/DT | DT |  | 125412 |

(continued)

| Animal # | Imm. Group | detecting antigen | IgG (H+L) ELISA Units | |
| --- | --- | --- | --- | --- |
| | | | prebleed | 23 weeks |
| pool | | | ≤4 | |
| | Geo Mean | | | 86528 |
| 5176 | CC/TT/DT | TT | | 21 |
| 5177 | CC/TT/DT | TT | | 30 |
| 5179 | CT/TT/DT | TT | | 342 |
| 5216 | CT/TT/DT | TT | | 36 |
| 5219 | CT/TT/DT | TT | | 30 |
| pool | | | ≤2 | |
| | Geo Mean | | | 47 |

Example 25

Transcutaneous immunization using CT induces potent immune responses. The immune response to an intramuscular injection and oral immunization was compared to transcutaneous immunization using CT as adjuvant and antigen. Twenty-five μg of CT (List Biologicals, catalog #101b) dissolved in saline was administered orally in 25 μl to BALB/c mice (n=5) using a 200 μl pipette tip. The mice readily swallowed the immunization solution. Twenty-five μl of 1 mg/ml CT in saline was administered on the ear as described to the group labeled transcutaneous. Twenty-five μg of CT in saline was injected IM into the anterior thigh in the group labeled intramuscular.

The mice injected IM with CT developed marked swelling and tenderness at the injection site and developed high levels of anti-CT antibodies. Mice immunized transcutaneously had no redness or swelling at the site of immunization and developed high levels of ant-CT antibodies. Mice immunized orally developed far lower levels of antibodies compared to the mice immunized transcutaneously. This indicates that oral immunization through grooming in the transcutaneously immunized mice does not account for the high levels of antibodies induced by transcutaneous immunization. Overall, the transcutaneous route of immunization is superior to either oral or IM immunization as high levels of antibodies are achieved without adverse reactions to the immunization.

Table 20. Kinetics of Cholera toxin antibody titers in animals immunized by the transcutaneous, oral, or intramuscular route

| Animal # | Immunization Route | IgG (H+L) ELISA Units | |
| --- | --- | --- | --- |
| | | prebleed | week 6 |
| 8962 | transcutaneous | | 23489 |
| 8963 | transcutaneous | | 30132 |
| 8964 | transcutaneous | | 6918 |
| 8965 | transcutaneous | | 20070 |
| 8825 | transcutaneous | | 492045 |
| pool | | 16 | |
| | Geo Mean | | 34426 |
| 8951 | oral | | 743 |
| 8952 | oral | | 4549 |
| 8953 | oral | | 11329 |
| 8954 | oral | | 1672 |
| pool | | 14 | |

(continued)

| Animal # | Immunization Route | IgG (H+L) ELISA Units | |
|---|---|---|---|
| | | prebleed | week 6 |
| | Geo Mean | | 2829 |
| 8955 | intramuscular | | 35261 |
| 8958 | intramuscular | | 607061 |
| 8959 | intramuscular | | 452966 |
| 8850 | intramuscular | | 468838 |
| 8777 | intramuscular | | 171648 |
| pool | | 12 | |
| | Geo Mean | | 239029 |

Example 26

BALB/c mice were immunized transcutaneously as described above for "Immunization Procedure", in groups of five mice. The mice were immunized at 0, 8 and 20 weeks using 100 µl of immunization solution prepared as follows: Hib conjugate (Connaught, lot #6J81401, 86 µg/ml) was lyophilized in order to concentrate the antigen. The lyophilized product was mixed in saline to make an immunizing solution containing 50 µg of Hib conjugate per 100 µl of saline for the group receiving Hib conjugate alone; Hib conjugate and CT were mixed in saline to make an immunizing solution containing 50 µg of Hib conjugate and 100 µg of CT per 100 µl of saline for the group receiving Hib conjugate and CT.

The antibodies were determined using ELISA as described above for "ELISA IgG(H+L)" on sera 3 weeks after the second immunization. The results are shown in Table 21. Hib conjugate alone induced a small but detectable antibody response. However, the addition of CT stimulated a far stronger immune response to Hib conjugate. CT acted as an adjuvant for the immune response to Hib conjugate. This indicates that a polysaccharide conjugate antigen can be used as a transcutaneous antigen by the method described.

Table 21. Antibody to Haemophilus influenzae b (Hib)

| Animal # | Imm. Group | IgG (H+L) ELISA Units |
|---|---|---|
| 5211 | Hib | 57 |
| 5212 | Hib | 29 |
| 5213 | Hib | 28 |
| 5214 | Hib | 63 |
| 5215 | Hib | 31 |
| | Geo Mean | 39 |
| 5201 | CT/Hib | 1962 |
| 5202 | CT/Hib | 3065 |
| 5203 | CT/Hib | 250 |
| 5204 | CT/Hib | 12 |
| 5205 | CT/Hib | 610 |
| | Geo Mean | 406 |
| pool prebleed | - | 1 |

Example 27

Emulsions, creams and gels may provide practical advantages for convenient spreading of the immunizing compound over the skin surface, over hair or body creases. Additionally such preparations may provide advantages such as occlusion or hydration which may enhance the efficiency of the immunization.

Heat labile entertoxin (LT) from *E. coli* (Sigma, catalog #E-8015, lot 17hH1200) was used to compare the efficiency of transcutaneous immunization using a simple saline solution and a commonly available petroleum base ointment, AQUAPHOR, which "can be used alone or in compounding virtually any ointment using aqueous solutions or in combination with other oil based substances and all common topical medications." (page 507 PDR, for Non-prescriptions Drugs, 1994, 15th Ed.). Mice were treated with a range of doses to evaluate the relative antibody response for the decreasing doses in the comparative vehicles.

BALB/c mice were immunized as described above except that the immunizing solution was applied for 3 hours on the back. Saline solutions of LT were prepared to deliver a 50 μl dose of solution and either 100 μg, 50 μg, 25 μg or 10 μg of antigen in the solution, using a 2 mg/ml, 1 mg/ml, 0.5 mg/ml or 0.2 mg/ml solution, respectively. After 3 hours the back was gently wiped using wetted gauze to remove the immunizing solution.

The water in oil preparation was performed as follows: equal volumes of AQUAPHOR and antigen in saline solution were mixed in 1 ml glass tuberculin syringes with luer locks using a 15 gauge emulsifying needle connecting the two syringes and mixing until the mixture was homogenous. A 4 mg/ml, 2 mg/ml, 1 mg/ml or 0.5 mg/ml solution of LT in saline was used, respectively, to mix with an equal volume of AQUAPHOR. 50 μl of this mixture was applied to the shaved back for three hours and then gently removed by wiping with gauze. Doses of antigen for the water in oil LT containing emulsions were weighed in order to deliver 50 μl. The weight per volume ratio was calculated by adding the specific gravity of saline (1.00 g/ml) and AQUAPHOR, 0.867 gm/ml, and dividing the sum by 2 for a final specific gravity of 0.9335 gm/ml. Approximately 47 mg of water in oil emulsion containing LT was delivered to the mouse for immunization.

A dose-response relationship was evident for both saline and water in oil emulsion delivered LT (Table 22). One hundred μg induced the highest level of antibodies and 10 μg induced a lower but potent immune response. Water in oil emulsified LT induced a similar response to LT in saline and appears to offer a convenient delivery mechanism for transcutaneous immunization. Similarly, gels, creams or more complex formulations such as oil-in water-in-oil could be used to deliver antigen for transcutaneous immunization. Such compositions could be used in conjunction with patches, occlusive dressings, or reservoirs and may allow long-term application or short term application of the immunizing antigen and adjuvant.

Table 22. Serum antibody against *E. coli* heat-labile enterotoxin (LT) in animals immunized with varying doses of LT in a saline or AQUAPHOR emulsion

| Imm | | IgG (H+L) Elisa Units | | | | IgG (H+L) Elisa Units | | |
|---|---|---|---|---|---|---|---|---|
| Group | emulsion | animal id# | pre-bleed | week 3 | emulsion | animal id# | pre-bleed | week 3 |
| LT 100 μg | saline | 8741 | | 18434 | aquaphor | 8717 | | 6487 |
| LT 100 μg | saline | 8742 | | 16320 | aquaphor | 8719 | | 4698 |
| LT 100 μg | saline | 8743 | | 19580 | aquaphor | 8774 | | 18843 |
| LT 100 μg | saline | 8744 | | 19313 | aquaphor | 8775 | | 18217 |
| LT 100 μg | saline | 8745 | | 22875 | aquaphor | 8861 | | 16230 |
| LT 100 μg | | pool | 32 | | | pool | 54 | |
| | | | | 19190 | | | | 11117 |
| Geo Mean | | | | | | | | |
| LT 50 μg | saline | 8736 | | 19129 | aquaphor | 8721 | | 4160 |
| LT 50 μg | saline | 8737 | | 3975 | aquaphor | 8722 | | 12256 |
| LT 50 μg | saline | 8738 | | 6502 | aquaphor | 8725 | | 12262 |
| LT 50 μg | saline | 8739 | | 6224 | aquaphor | 8771 | | 12982 |

(continued)

| Imm | | IgG (H+L) Elisa Units | | | IgG (H+L) Elisa Units | |
|---|---|---|---|---|---|---|
| LT 50 μg | saline | 8740 | 18449 | aquaphor | 8772 | 15246 |
| LT 50 μg | pool | 54 | | pool | 57 | |
| | | | 8929 | | | 10435 |
| Geo Mean | saline | 8768 | 3274 | aquaphor | 8727 | 3585 |
| | saline | 8731 | 3622 | aquaphor | 8728 | 3 |
| | saline | 8732 | 557 | aquaphor | 8729 | 4206 |
| LT 25 μg | saline | 8733 | 626 | aquaphor | 8862 | 7353 |
| LT 25 μg | saline | 8734 | 1725 | aquaphor | 8769 | 5148 |
| LT 25 μg | pool | 56 | | pool | 53 | |
| LT 25 μg | | | | | | |
| LT 25 μg | | | 1481 | | | 1114 |
| | saline | 8848 | 621 | aquaphor | 8748 | 1968 |
| Geo Mean | saline | 8849 | 475 | aquaphor | 8749 | 1935 |
| | saline | 8757 | 858 | aquaphor | 8750 | 646 |
| LT 10 μg | saline | 8759 | 552 | aquaphor | 8747 | 1569 |
| LT 10 μg | saline | 8760 | 489 | aquaphor | 8764 | 1 |
| LT 10 μg | pool | 43 | | pool | 39 | |
| LT 10 μg | | | 585 | | | 329 |
| LT 10 μg | | | | | | |
| Geo Mean | | | | | | |

Example 28

Mice were immunized transcutaneously as described above for "Immunization Procedure", in groups of five mice. Mice were immunized at 0, 8 and 18 weeks with 100 μl saline containing 50 μg Tetanus toxoid (List Biologicals, catalog #191B, lots #1913a and #1915b) and 83 μg Diphtheria toxoid (List Biologicals, catalog #151, lot #15151) alone or in combination with 100 μg Cholera toxin (List Biologicals, catalog #101B, lot #10149CB).

Anti-Diphtheria toxoid antibodies were quantitated using ELISA as described for "ELISA IgG (H+L)". Elevated levels of anti-toxoid antibodies were detected in animals given immunized with either TT/DT or CT/TT/DT. However, the antibody titers were far superior in animals in which CT was included as an adjuvant. This anti-Toxoid titer was obviously increased in both groups after each subsequent immunization (8 and 18 weeks). Thus while DT can induce a small but significant response against itself the magnitude of the response can be increased by 1) inclusion of cholera toxin as an adjuvant and 2) boosting with the adjuvant Cholera toxin and antigen (Diphtheria toxoid). Classic boosting responses are dependent on T-cell memory and the boosting of the anti-DT antibodies in this experiment indicate that T-cells are engaged by transcutaneous immunization.

Table 23. Kinetics of Diphtheria toxoid (DT) antibody titers in animals immunized with Tetanus toxoid (TT) and DT or Cholera toxin (CT), TT, and DT

| Animal # | Immunization Group | Detecting Antigen | prebleed | week 2 | week 4 | week 6 | week 8 | week 10 | week 14 | week 17 | week 18 | week 20 | week 23 |
|---|---|---|---|---|---|---|---|---|---|---|---|---|---|
| | | | | | | | | | | | | IgG (H+L) ELISA Units | | |
| 5196 | TT/DT | DT | | 7 | 12 | 18 | 23 | 49 | 56 | 33 | 18 | 219 | 166 |
| 5197 | TT/DT | DT | | 5 | 11 | 11 | 10 | 15 | 17 | 16 | 17 | 125 | 75 |
| 5198 | TT/DT | DT | | 13 | 20 | 16 | - | 28 | 25 | 27 | 7 | 48 | 172 |
| 5199 | TT/DT | DT | | 13 | 8 | 10 | 10 | 11 | 22 | 12 | 217 | 178 | 263 |
| 5200 | TT/DT | DT | | 4 | 10 | 4 | 7 | 120 | 149 | 127 | - | 17309 | 14537 |
| | | Geo Mean | | 7 | 12 | 10 | 11 | 31 | 38 | 29 | 26 | 332 | 382 |
| 5176 | CT/TT/DT | DT | | 8 | 26 | 21 | 14 | 3416 | 5892 | 1930 | 1826 | 63087 | 64704 |
| 5177 | CT/TT/DT | DT | | 8 | 6 | 7 | 8 | 424 | 189 | 149 | 175 | 16416 | 17941 |
| 5179 | CT/TT/DT | DT | | 8 | 3 | 4 | 8 | 4349 | 1984 | 2236 | 1921 | 124239 | 114503 |
| 5216 | CT/TT/DT | DT | | 12 | 5 | 9 | 11 | 3238 | 2896 | 2596 | 1514 | 278281 | 290964 |
| 5219 | CT/TT/DT | DT | | 8 | 15 | 13 | 12 | 5626 | 4355 | 2036 | 1941 | 343161 | 125412 |
| | | Geo Mean | | 9 | 8 | 9 | 10 | 2582 | 1945 | 1277 | 1125 | 104205 | 86528 |
| pool | | | 4 | | | | | | | | | | |

EP 1 384 403 B1

Example 29

C57B1/6 mice were immunized transcutaneously with CT (azide-free, Calbiochem) as described above on the shaved back of the mouse. Mice were challenged using a lethal challenge model 6 weeks after immunization (Mallet et al, Immunoprophylactic efficacy of nontoxic mutants of *Vibrio cholerae* toxin (CTK63) and *Escherichia coli* heat-labile toxin (LTK63) in a mouse cholera toxin intranasal challenge model, submitted to *Immunology Letters*). In the challenge, mice were given 20 μg of CT (Calbiochem, azide free) dissolved in saline intranasally via a plastic pipette tip while anesthetized with 20 μl of ketamine-rompin. In trial #1, 12/15 immunized mice survived the challenge after 14 days and 1/9 unimmunized control mice survived. Five control mice were lost prior to challenge due to anesthesia. Mice in challenge #1 had anti-CT serum antibodies of 15,000 ELISA units (geometric mean), and five immunized mice sacrificed at the time of challenge had lung wash IgG detected in 5/5 mice. Lung washes were collected as described above.

The immunization and challenge was repeated with naive C57B1/6 mice and 7/16 immunized mice survived the challenge, while only 2/17 unimmunized mice survived the challenge. Immunized mice in challenge #2 had anti-CT IgG antibodies of 41,947 ELISA units (geometric mean). Lung washes from five mice sacrificed at the time of challenge demonstrated both anti-CT IgG and IgA (Table 24). Stool samples from 8/9 mice demonstrated both anti-CT IgG and IgA (Table 25). Stool samples were collected fresh from animals spontaneously defecating at the time of challenge. The stools were frozen at -20°C. At the time of ELISA, the stools were thawed, homogenized in 100 μl of PBS, centrifuged and ELISA run on the supernatant. The combined survival rate among immunized mice was 19/31 or 61% whereas the combined survival rate among unimmunized mice was 3/26 or 12%.

Table 24. Lung wash anti-Cholera toxin IgG and IgA antibody titers

| Sample Dilution | Animal Identification Number | | | | |
|---|---|---|---|---|---|
| | 8969 | 8970 | 8971 | 8972 | 8995 |
| IgG (H+L) anti-CT (Optical Density) | | | | | |
| 1:10 | 3.613 | 3.368 | 3.477 | 3.443 | 3.350 |
| 1:20 | 3.302 | 3.132 | 3.190 | 3.164 | 3.166 |
| 1:40 | 3.090 | 2.772 | 2.825 | 2.899 | 2.692 |
| 1:80 | 2.786 | 2.287 | 2.303 | 2.264 | 2.086 |
| 1:160 | 2.041 | 1.570 | 1.613 | 1.624 | 1.441 |
| 1:320 | 1.325 | 0.971 | 1.037 | 1.041 | 0.965 |
| 1:640 | 0.703 | 0.638 | 0.601 | 0.644 | 0.583 |
| 1:1280 | 0.434 | 0.382 | 0.350 | 0.365 | 0.364 |
| IgA anti-CT (Optical Density) | | | | | |
| 1:2 | 1.235 | 2.071 | 2.005 | 2.115 | 1.984 |
| 1:4 | 1.994 | 1.791 | 1.836 | 1.85 | 1.801 |
| 1:8 | 1.919 | 1.681 | 2.349 | 1.796 | 1.742 |
| 1:16 | 1.8 | 1.457 | 1.577 | 1.614 | 1.536 |
| 1:32 | 1.503 | 1.217 | 1.36 | 1.523 | 1.23 |
| 1:64 | 1.189 | 0.863 | 1.044 | 1.101 | 0.88 |
| 1:128 | 0.814 | 0.57 | 0.726 | 0.74 | 0.595 |
| 1:356 | 0.48 | 0.334 | 0.436 | 0.501 | 0.365 |

Table 25. Stool anti-Cholera toxin IgG and IgA antibody titers

| Sample Dilution | 8985 (CT) | 8997 (CT) | 8987 (CT) | 8990 (CT) | 8977 (CT) | 8976 (CT) | 8975 (CT) | 8988 (CT) | 8994 (none) | 8979 (none) | 9000 (none) | 8983 (none) |
|---|---|---|---|---|---|---|---|---|---|---|---|---|
| mouse identification number (immunization group) | | | | | | | | | | | | |
| IgG (H+L) anti-CT (optical density) | | | | | | | | | | | | |
| 1:10 | 1.01 | 1.91 | 2.33 | 0.03 | 0.74 | 1.98 | 1.20 | 1.45 | 0.09 | 0.05 | 0.02 | 0.18 |
| 1:20 | 0.42 | 0.94 | 1.26 | - | 0.31 | 1.19 | 0.50 | 0.91 | 0.04 | - | - | 0.08 |
| 1:40 | 0.20 | 0.46 | 0.68 | - | 0.12 | 0.58 | 0.24 | 0.49 | - | - | - | 0.02 |
| 1:80 | 0.10 | 0.21 | 0.34 | - | 0.05 | 0.31 | 0.09 | 0.25 | - | - | - | - |
| 1:160 | 0.03 | 0.09 | 0.18 | - | 0.02 | 0.14 | 0.05 | 0.12 | - | - | - | - |
| IgA Anti-CT (optical density) | | | | | | | | | | | | |
| 1:4 | 0.32 | 1.14 | 0.43 | 0.00 | 0.19 | 1.00 | 0.58 | 1.21 | 0.02 | - | 0.07 | - |
| 1:8 | 0.16 | 0.67 | 0.24 | - | 0.08 | 0.56 | 0.36 | 0.77 | - | - | - | - |
| 1:16 | 0.08 | 0.33 | 0.11 | - | 0.03 | 0.27 | 0.17 | 0.40 | - | - | - | - |
| 1:32 | 0.06 | 0.16 | 0.05 | - | 0.03 | 0.12 | 0.08 | 0.20 | - | - | - | - |
| 1:64 | 0.01 | 0.07 | 0.03 | - | - | 0.05 | 0.03 | 0.10 | - | - | - | - |

Example 30

C57B1/6 female mice were obtained from Charles River Laboratories. The mice were immunized with 200 $\mu$g ovalbumin (OVA) (Sigma, lot #14H7035, stock concentration of 2 mg/ml in PBS) and 50 $\mu$g Cholera Toxin (List Biologicals, lot #101481B, stock concentration of 5 mg/ml). A Packard Cobra Gamma Counter was used (serial #102389) to measure the amount of $^{51}$Cr released.

C57B1/6 mice were anesthetized with 0.03 ml of ketamine-rompin and shaved on the dorsum with a clipper, without traumatizing the skin, and were rested for 24 hours. The mice were anesthetized then immunized at 0 and 28 days with 150 $\mu$l of immunizing solution placed on the shaved skin over a 2 cm$^2$ area for 2 hours. The mice were then wiped twice with wet gauze. The mice exhibited no adverse effects from either anesthesia, immunization, or the washing procedure. This procedure was repeated weekly for three weeks.

Splenic lymphocytes were collected one week after boosting immunization. Cells were cultured in vitro in RPMI-1640 and 10% FBS (with penicillinstreptomycin, glutamine, non-essential amino acids, sodium pyruvate and 2-mercaptoethanol) for 6 days with the addition of 5% rat concanavalin A supernatant as a source of IL-2, with or without antigen. Target cells consist of syngeneic (H-2$^b$) EL4 cells alone and EL4 cells pulsed with the CTL peptide SINFEKKL, allogeneic (H-2k) L929 cells and EG7 cells. The target cells (1 x 10$^6$ cells per well) were labeled for 1 h with 0.1 mCi per well $^{51}$Cr (Na$_2$CrO$_4$ source, Amersham) and were added to effector cells at ratios ranging from 3:1 to 100:1. The cell mixtures were incubated in 96-well round bottom tissue culture plates (Costar, catalog #3524) in 0.2 ml complete RPMI-1640, 10% FBS medium for 5 h at 37°C in a 5% CO$_2$ humidified atmosphere. At the end of the 5 h culture, the supernatants were absorbed by cotton wicks and processed for the determination of 51Cr release. Specific lysis was determined as:

$$\texttt{\% Specific Lysis = 100 x [(experimental release - spontaneous release) / (maximal release - spontaneous release)].}$$

As shown in Table 26, part 1 CTLs were detected against the EL4 peptide pulsed cells at an E:T ratio of 100:1 for the group immunized with CT+OVA. CTL assays are not positive if the percent specific lysis is not above 10% or clearly above the media-stimulated effectors background percentage lysis. Similarly, as shown in Table 26, part 2 CTLs were detected against the EG7 (OVA transfected cells) at an E:T ratio of 100:1 for the group immunized with CT+OVA. Thus, CT adjuvanted for the production of CTLs via the transcutaneous route.

Table 26. OVA-specific CTL induced Transcutaneously

Part 1 - Target Cells: EL4+Peptide

| | Imm. Group | | | | | |
| | CT+OVA | CT+OVA | CT | CT | OVA | OVA |
| | Stimulated with | | | | | |
| E:T Ratio | Media | Ova | Media | Ova | Media | Ova |
| 100:1 | 9.5 | 13.1 | 11.1 | 12.5 | 23.1 | 21.5 |
| 30:1 | 6.9 | 6.8 | 5.9 | 8.9 | 14.2 | 10.7 |
| 10:1 | 4.9 | 3.5 | 3.5 | 8.5 | 7.7 | 5.2 |

Part 2 - Target Cells: EG7 (OVA Transfected)

| | Imm. Group | | | | | |
| | CT+OVA | CT+OVA | CT | CT | OVA | OVA |
| | Stimulated with | | | | | |
| E:T Ratio | Media | Ova | Media | Ova | Media | Ova |
| 100:1 | 10.6 | 17.6 | 14.5 | 16.8 | 23.8 | 26 |
| 30:1 | 4.9 | 9.5 | 8.2 | 10.1 | 13.6 | 10.7 |
| 10:1 | 6.4 | 4.4 | 4 | 5 | 7.3 | 4.2 |

Example 31

C57B1/6 mice (n=6) were immunized transcutaneously as described above for "Immunization Procedure". Mice were immunized at 0 and 4 weeks with 100 $\mu$l saline containing 100 $\mu$g Cholera toxin (List Biologicals, catalog #101B, lot #10149CB) and 250 $\mu$g of ovalbumin protein (Sigma, albumin chicken egg, Grade V catalog #A5503, lot #14H7035).

Single cell suspensions were prepared from spleens harvested from animals at eight weeks after the first immunization. Splenocytes were set up in culture at 8 x 10⁵ cells per well in a 200 $\mu$l volume containing OVA protein or the irrelevant protein Conalbumin at the concentrations indicated. Cultures were incubated for 72 hours at 37°C in a $CO_2$ incubator at which time 0.5 $\mu$Ci/well of ³H thymidine was added to each well. Twelve hours later, proliferation was assessed by harvesting the plates and quantitating incorporated radiolabelled thymidine by liquid scintillation counting. Raw values of ³H incorporation are indicated in cpm and the fold increase (cpm experimental / cpm media) is indicated to the left of each sample. Fold increases greater than three are considered significant.

Significant proliferation was only detected when the splenocytes were stimulated with the protein, Ovalbumin, to which the animals had been immunized with in vivo and not with the irrelevant protein conalbumin. Thus transcutaneous immunization with Cholera toxin and ovalbumin protein induces antigen specific proliferation of splenocytes in vitro indicating that a cellular immune response is evoked by this method.

Table 27. Antigen specific proliferation of spleen cells from animals immunized with Cholera toxin (CT) and Ovalbumin (OVA)

| Immunization group Conalbumin | Concentration media of *in vitro* stimuli | | OVA protein | |
|---|---|---|---|---|
| | 3-H incorporation cpm | fold increase | 3-H incorporation cpm | fold increase |
| CT/OVA | 10 $\mu$g/ml1427    13450 | 9.4 | 3353 | 2.3 |
| | 1 $\mu$g/ml    4161 | 2.9 | 2638 | 1.8 |
| | 0.1 $\mu$g/ml    2198 | 1.5 | 2394 | 1.7 |
| | 0.01 $\mu$g/ml    3419 | 2.4 | 2572 | 1.8 |

REFERENCES

Aiba and Katz (1990) Immunology 145:2791-2796
Alving et al (1986) Vaccine 4:166-172.
Alving and Wassef (1994) AIDS Res Hum Retro 10 (suppl. 2):S91-S94.
Alving et al (1993) In: *Liposome Technology,* vol. 3, pp. 317-343.
Antel et al (1996) Nature Medicine 2:1074-1075. Ausubel et al (1996) *Current Protocols in Molecular Biology.*
Bacci et al (1997) Eur J Immunol 27:442-448.
Bathurst et al (1993) Vaccine 11:449-456. Bellinghausen et al (1996) J Invest Dermatol 107:582-588.
Blauvelt et al (1995) J Invest Dermatol 104:293-296. Blum (1995) Digestion 56:85-95.
Bodanszky (1993) Peptide *Chemistry.*
Bos (1997) Clin Exp Immunol 107 (suppl. 1):3-5.
Bowen et al (1994) Immunology 81:338-342.
Burnette et al (1994) In: *Bioprocess Technology,* pp. 185-203.
Caughman et al (1986) Proc Natl Acad Sci USA 83:7438-7442.
Chang et al (1989) Proc Natl Acad Sci USA 86:6343-6347.
Chang et al (1992) J Immunol 139:548-555.
Chang et al (1994) J Immunol 152:3483-3490.
Craig and Cuatrecasas (1975) Proc Natl Acad Sci USA 72:3384-2288.
Dahl (1996) In: *Clinical Immunodermatology,* 3rd Ed., pp. 345-352.
Delenda et al (1994) J Gen Virol 75:1569-1578.
Deprez et al (1996) Vaccine 14:375-382.
Deutscher (1990) Guide to *Protein Purification.* Dragunsky et al (1992) Vaccine 10:735-736.
Elson and Dertzbaugh (1994) In: *Handbook of Mucosal Immunology,* p. 391.
Enk et al (1993) J Immunol 150:3698-3704.
Fonseca et al (1994) Vaccine 12:279-285.
Frankenburg et al (1996) Vaccine 14:923-929.
Fries et al (1992a) Proc Natl Acad Sci USA 89:358-362.

Fries et al (1992b) Infect Immunity 60:1834-1839.

Glenn et al (1995) Immunol Lett 47:73-78.

Goeddel (1990) *Gene Expression Technology.*

Gregoriadis (1993) *Liposome Preparation and Related Techniques,* 2nd Ed.

Herrington et al (1991) Am J Trop Med Hyg 45:695-701.

Jahrling et al (1996) Arch Virol Suppl 11:135-140.

Janeway and Travers (1996) *Immunobiology.*

Janson and Ryden (1989) *Protein Purification.*

Katkov (1996) Med Clin North Am 80:189-200.

Khusmith et al (1991) Science 252:715-718.

Kounnas et al (1992) J Biol Chem 267:12420-12423.

Kriegler (1990) *Gene Transfer and Expression.*

Kripke et al (1990) J Immunol 145:2833-2838.

Krueger and Barbieri (1995) Clin Microbiol Rev 8:34-47.

Lee and Chen (1994) Infect Immunity 62:3594-3597.

Leung (1997) Clin Exp Immunol 107 (Suppl. 1):25-30.

Lycke and Holmgren (1986) Immunology 59:301-308.

Lieberman and Greenberg (1996) Adv Pediatr Infect Dis 11:333-363.

Malik et al (1991) Proc Natl Acad Sci USA 88:3300-3304.

Mast and Krawczynski (1996) Annu Rev Med 47:257-266.

Migliorini et al (1993) Eur J Immunol 23:582-585.

Morein and Simons (1985) Vaccine 3:83-93.

Murray (1991) *Gene Transfer and Expression Protocols.*

Nohria and Rubin (1994) Biotherapy 7:261-269.

Paul and Cevc (1995) Vaccine Res 3:145-164.

Paul et al (1995) Eur J Immunol 25:3521-3524, 1995.

Paul and Seder (1994) Cell 76:241-251.

Peguet-Navarro et al (1995) Adv Exp Med Biol 378:359-361.

Pessi et al (1991) Eur J Immunol 24:2273-2276.

Pierce (1978) J Exp Med 148:195-206.

Pierce and Reynolds (1974) J Immunol 113:1017-1023.

Plotkin and Mortimer (1994) *Vaccines.*

Porgador et al (1997) J Immunol 158:834-841.

Rappuoli et al (1995) Int Archiv Allergy Immunol 108:327-333.

Rappuoli et al (1996) Adv Exp Med Biol 397:55-60.

Ribi et al (1988) Science 239:1272-1276.

Richards et al (1995) In: *Vaccine Design.*

Roberts and Walker (1993) In: *Pharmaceutical Skin Penetration Enhancement.*

Saloga et al (1996) Exp Dermatol 5:65-71.

Schneerson et al (1996) Lancet 348:1289-1292.

Schuler and Steinman (1985) J Exp Med 161:526-546.

Schwarzenberger and Udey (1996) J Invest Dermatol 106:553-558.

Scopes (1993) *Protein Purification.*

Seder and Paul (1994) Annu Rev Immunol 12:635-673.

Shafara et al (1995) Ann Intern Med 125:658-668.

Shankar et al (1996) Cell Immunol 171:240-245.

Skeiky et al (1995) J Exp Med 181:1527-1537.

Smedile et al (1994) Prog Liver Dis 12:157-175.

Smucny et al (1995) Am J Trop Med Hyg 53:432-437.

Stacey et al (1996) J Immunol 157:2116-2122.

Stingl et al (1989) Immunol Ser 46:3-42.

Streilein and Grammer (1989) J Immunol 143:3925-3933.

Summers and Smith (1987) *A Manual of Methods for Baculovirus Vectors and Insect Cell Culture Procedure.*

Tam (1988) Proc Natl Acad Sci USA 85:5409-5413.

Tew et al (1997) Immunol Rev 156:39-52.

Trach et al (1997) Lancet 349:231-235.

Udey (1997) Clin Exp Immunol 107 (Suppl. 1):6-8.

Vandenbark et al (1996) Nature Medicine 2:1109-1115.

Vreden et al (1991) Am J Trop Med Hyg 45:533-538.
Wang et al (1995) J Immunol 154:2784-2793.
Wertz (1992) In: *Liposome Dramatics,* pp. 38-43.
White et al (1993) Vaccine 11:1341-1346.
Wiesmueller et al (1991) Immunology 72:109-113.
Wisdom (1994) *Peptide Antigens.*
Zhang et al (1995) Infect Immun 63:1349-1355.

**Claims**

1. Use of an antigen and an adjuvant, in the manufacture of a formulation for transcutaneous immunization of a subject wherein said formulation induces an immune response specific for the antigen following application of the formulation to the subject's skin, and further wherein the formulation does not include a penetration enhancer.

2. The use of claim 1, wherein the adjuvant is selected from the group consisting of bacterial DNA, cytokines, chemokines and lipopolysaccharides, a molecule containing unmethylated CpG motifs, a heat shock protein, a derivative of a heat shock protein and combinations thereof.

3. The use of claim 1, wherein the formulation further comprises a dressing to form a patch for transcutaneous immunization.

4. The use of claim 3, wherein the dressing is an occlusive dressing.

5. The use of claim 1, wherein an amount of antigen effective to induce the immune response is not encapsulated in a liposome.

6. The use of claim 1, wherein the adjuvant enhances presentation of the antigen to a lymphocyte.

7. The use of claim 1, wherein the adjuvant activates an antigen presenting cell.

8. The use of claim 7, wherein the antigen presenting cell is a Langerhans cell or a dermal dendritic cell.

9. The use of claim 1, wherein the adjuvant increases major histocompatibility complex class II expression on an antigen presenting cell.

10. The use of claim 9, wherein the antigen presenting cell is a Langerhans cell or a dermal dendritic cell.

11. The use of claim 1, wherein the adjuvant causes an antigen presenting cell underlying the application site to migrate to a draining lymph node.

12. The use of claim 11, wherein the antigen presenting cell is a Langerhans cell or a dermal dendritic cell.

13. The use of claim 1, wherein the adjuvant signals a Langerhans cell to mature into a dendritic cell.

14. The use of claim 1, wherein the formulation consists essentially of antigen and adjuvant.

15. The use of claim 1, wherein a single molecule acts as both antigen and adjuvant.

16. The use of claim 15, wherein the formulation consists essentially of only one component which is both an antigen and an adjuvant.

17. The use of claim 1, wherein the antigen lacks adjuvant properties.

18. The use of claim 1, wherein the formulation is an aqueous solution.

19. The use of claim 1, wherein the formulation does not include an organic solvent.

20. The use of claim 1, wherein the formulation is formed as a cream, an emulsion, a gel, a lotion, an ointment, a paste or a suspension.

21. The use of claim 1, wherein the antigen is at least partially purified and derived from a pathogen selected from the group consisting of bacterium, virus, fungus and parasite.

22. The use of claim 1, wherein the antigen is an at least partially purified tumor antigen.

23. The use of claim 1, wherein the antigen is an at least partially purified autoantigen.

24. The use of claim 1, wherein the antigen is an at least partially purified allergen.

25. The use of claim 1, wherein the antigen is at least partially purified and greater than 500 daltons molecular weight.

26. The use of claim 1, wherein the antigen is at least partially purified and greater than 800 daltons molecular weight.

27. The use of claim 1, wherein the antigen is at least partially purified and greater than 1000 daltons molecular weight.

28. The use of any one of claims 25-27, wherein the antigen is proteinaceous.

29. The use of any one of claims 25-27, wherein the antigen is a conjugate with polysaccharide.

30. The use of claim 1, wherein the antigen is provided as a whole cell, a bacterium, a virion, a live virus, an attenuated virus, an inactivated virus or a virosome.

31. The use of claim 30, wherein the virus is rabies virus.

32. The use of claim 30, wherein the virus is influenza virus.

33. The use of claim 30, wherein the bacterium is anthrax.

34. The use of claim 30, wherein the bacterium is enterotoxigenic *E. coli.*

35. The use of claim 1, wherein the formulation includes at least two different antigens.

36. The use of claim 1, wherein the antigen is provided as a nucleic acid comprising a sequence encoding the antigen, the adjuvant is provided as a nucleic acid comprising a sequence encoding the adjuvant, or both the antigen and adjuvant are provided as a nucleic acid comprising sequences encoding each.

37. The use of claim 36, wherein the nucleic acid is non-integrating and non-replicating.

38. The use of claim 36, wherein the nucleic acid further comprises a regulatory region operably linked to the sequence encoding the antigen and/or adjuvant.

39. The use of claim 36, wherein the formulation does not include a viral article, liposomes or charged lipid.

40. The use of claim 1, further comprising the use of a hydrating agent.

41. The use of claim 1, further comprising the use of a humectant.

42. The use of claim 41, wherein the humectant is glycerol.

43. The use of claim 1, wherein the formulation comprises an amount of antigen effective to induce the immune response, which antigen is in solution and/or associated with, but not encapsulated in, a liposome.

44. The use of claim 1, wherein the antigen is carbohydrate, glycolipid, glycoprotein, lipid, lipoprotein, phospholipid, polypeptide, protein, or conjugates thereof.

**45.** The use of any one of claims 1-44, wherein the adjuvant binds a receptor on antigen presenting cells.

**46.** The use of any one of claims 1-44, wherein the adjuvant binds $GM_1$-ganglioside.

**47.** The use of any one of claims 1-44, wherein the adjuvant binds $\alpha_2$- macroglobulin receptor-low density lipoprotein receptor-related protein.

**48.** The use of any one of clams 1-44, wherein the immune response is not an allergic reaction, dermatitis or an atopic reaction.

**49.** A patch for use in transcutaneous immunisation, the patch comprising an antigen and an adjuvant, wherein application of the patch to a subject's skin induces an immune response specific for the antigen.

**50.** A patch for use in any one of claims 1-48.

**51.** The patch of claim 50, wherein application of said patch hydrates the formulation.

**52.** The use of any one of claims 1-20, 28, 29, 35 and 40-44, wherein the antigen is expressed by recombinant means.

**53.** The use of claim 52, wherein the recombinantly expressed antigen is a fusion protein comprising an affinity tag or an epitope tag.

**54.** The use of claim 30, wherein the virus is adenovirus.

**55.** The use of claim 30, wherein the virus is selected from the group consisting of hepatitis serotype A, B, C, D, E or combinations thereof.

**56.** The use of claim 30, wherein the virus is human immunodeficiency virus.

**57.** The use of claim 30, wherein the virus is papilloma virus.


**Patentansprüche**

**1.** Verwendung eines Antigens und eines Adjuvans bei der Herstellung einer Formulierung für die transkutane Immunisierung eines Probanden, wobei die Formulierung eine Immunantwort auslöst, die spezifisch für das Antigen ist, und die auf die Anwendung der Formulierung auf der Haut des Probanden folgt, und wobei die Formulierung darüber hinaus keinen Penetrationsverstärker umfasst.

**2.** Verwendung nach Anspruch 1, wobei das Adjuvans aus der Gruppe ausgewählt wird, die aus einer bakteriellen DNA, Cytokinen, Chemokinen und Lipopolysacchariden, einem Molekül, das unmethylierte CpG-Motive enthält, einem Hitzeschockprotein, einem Derivat eines Hitzeschockproteins und Kombinationen derselben besteht.

**3.** Verwendung nach Anspruch 1, wobei die Formulierung darüber hinaus eine Zubereitung umfasst, um ein Pflaster für die transkutane Immunisierung zu bilden.

**4.** Verwendung nach Anspruch 3, wobei die Zubereitung eine eingeschlossene Zubereitung ist.

**5.** Verwendung nach Anspruch 1, wobei eine Menge eines Antigens, die für die Auslösung der Immunantwort wirksam ist, nicht in einem Liposom verkapselt ist.

**6.** Verwendung nach Anspruch 1, wobei das Adjuvans die Präsentation des Antigens gegenüber einem Lymphocyten verstärkt.

**7.** Verwendung nach Anspruch 1, wobei das Adjuvans eine antigen-präsentierende Zelle aktiviert.

**8.** Verwendung nach Anspruch 7, wobei die antigen-präsentierende Zelle eine Langerhans-Zelle oder eine dermale dentritische Zelle ist.

9. Verwendung nach Anspruch 1, wobei das Adjuvans die Expression des Haupt-Histokompatibilitäts-Komplexes der Klasse II auf der antigen-präsentierenden Zelle steigert.

10. Verwendung nach Anspruch 9, wobei die antigen-präsentierende Zelle eine Langerhans-Zelle oder eine dermale dendritische Zelle ist.

11. Verwendung nach Anspruch 1, wobei das Adjuvans eine antigen-präsentierende Zelle, welche sich an der Stelle der Anwendung befindet, dazu veranlasst, zu einem drainierenden Lymphknoten zu wandern.

12. Verwendung nach Anspruch 11, wobei die antigen-präsentierende Zelle eine Langerhans-Zelle oder eine dermale dendritische Zelle ist.

13. Verwendung nach Anspruch 1, wobei das Adjuvans einer Langerhans-Zell signalisiert, zu einer dendritischen Zelle zu reifen.

14. Verwendung nach Anspruch 1, wobei die Formulierung im Wesentlichen aus dem Antigen und dem Adjuvans besteht.

15. Verwendung nach Anspruch 1, wobei ein einzelnes Molekül sowohl als Antigen als auch als Adjuvans wirkt.

16. Verwendung nach Anspruch 15, wobei die Formulierung im Wesentlichen aus lediglich einem Bestandteil besteht, der sowohl ein Antigen als auch ein Adjuvans ist.

17. Verwendung nach Anspruch 1, wobei dem Antigen die Eigenschaften des Adjuvans fehlen.

18. Verwendung nach Anspruch 1, wobei die Formulierung eine wässrige Lösung ist.

19. Verwendung nach Anspruch 1, wobei die Formulierung kein organisches Lösungsmittel umfasst.

20. Verwendung nach Anspruch 1, wobei die Formulierung als eine Creme, eine Emulsion, ein Gel, eine Lotion, eine Salbe, eine Paste oder eine Suspension formuliert ist.

21. Verwendung nach Anspruch 1, wobei das Antigen zumindest teilweise gereinigt ist und von einem Pathogen stammt, das aus der Gruppe ausgewählt wird, welche aus einem Bakterium, einem Virus, einem Pilz oder einem Parasiten besteht.

22. Verwendung nach Anspruch 1, wobei das Antigen ein mindestens teilweise gereinigtes Tumorantigen ist.

23. Verwendung nach Anspruch 1, wobei das Antigen ein mindestens teilweise gereinigtes Autoantigen ist.

24. Verwendung nach Anspruch 1, wobei das Antigen ein mindestens teilweise gereinigtes Allergen ist.

25. Verwendung nach Anspruch 1, wobei das Antigen mindestens teilweise gereinigt ist und ein Molekulargewicht von mehr als 500 Dalton aufweist.

26. Verwendung nach Anspruch 1, wobei das Antigen mindestens teilweise gereinigt ist und ein Molekulargewicht von mehr als 800 Dalton aufweist.

27. Verwendung nach Anspruch 1, wobei das Antigen mindestens teilweise gereinigt ist und ein Molekulargewicht von mehr als 1000 Dalton aufweist.

28. Verwendung nach einem der Ansprüche 25 - 27, wobei das Antigen proteinartig ist.

29. Verwendung nach einem der Ansprüche 25 - 27, wobei das Antigen ein Konjugat mit einem Polysaccharid ist.

30. Verwendung nach Anspruch 1, wobei das Antigen als eine ganze Zelle, ein Bakterium, ein Virion, ein lebender Virus, ein attenuierter Virus, ein inaktivierter Virus oder ein Virosom bereitgestellt wird.

31. Verwendung nach Anspruch 30, wobei der Virus ein Tollwut-Virus ist.

**32.** Verwendung nach Anspruch 30, wobei der Virus ein Influenza-Virus ist.

**33.** Verwendung nach Anspruch 30, wobei das Bakterium ein Anthraxbakterium ist.

**34.** Verwendung nach Anspruch 30, wobei das Bakterium ein enterotoxigenes E. coli ist.

**35.** Verwendung nach Anspruch 1, wobei die Formulierung mindestens zwei verschiedene Antigene umfasst.

**36.** Verwendung nach Anspruch 1, wobei das Antigen als eine Nukleinsäure bereitgestellt wird, die eine für das Antigen codierende Sequenz umfasst, wobei das Adjuvans als eine Nukleinsäure bereitgestellt wird, die eine für das Adjuvans codierende Sequenz umfasst, oder wobei sowohl das Antigen als auch das Adjuvans als eine Nukleinsäure bereitgestellt werden, die für das jeweilige Molekül codierende Sequenzen umfasst.

**37.** Verwendung nach Anspruch 36, wobei die Nukleinsäure nicht integrierend und nicht replizierend ist.

**38.** Verwendung nach Anspruch 36, wobei die Nukleinsäure darüber hinaus einen regulatorischen Bereich umfasst, der in funktioneller Weise mit der für das Antigen und/oder das Adjuvans codierenden Sequenz verbunden ist.

**39.** Verwendung nach Anspruch 36, wobei die Formulierung kein virales Teilchen, Liposom oder geladenes Lipid umfasst.

**40.** Verwendung nach Anspruch 1, welche darüber hinaus die Verwendung eines hydratisierenden Mittels umfasst.

**41.** Verwendung nach Anspruch 1, welche darüber hinaus die Verwendung eines Feuchthaltemittels umfasst.

**42.** Verwendung nach Anspruch 41, wobei das Feuchthaltemittel Glycerin ist.

**43.** Verwendung nach Anspruch 1, wobei die Formulierung eine Menge eines Antigens umfasst, die für die Auslösung einer Immunantwort wirksam ist, wobei das Antigen sich in Lösung befindet und / oder mit einem Liposom in Zusammenhang steht, jedoch nicht darin verkapselt ist.

**44.** Verwendung nach Anspruch 1, wobei das Antigen ein Kohlenhydrat, Glycolipid, Glycoprotein, Lipid, Lipoprotein, Phospholipid, Polypeptid, Protein oder Konjugat derselben ist.

**45.** Verwendung nach einem der Ansprüche 1 - 44, wobei das Adjuvans einen Rezeptor oder eine antigen-präsentierende Zelle bindet.

**46.** Verwendung nach einem der Ansprüche 1 - 44, wobei das Adjuvans ein GM1-Gangliosid bindet.

**47.** Verwendung nach einem der Ansprüche 1 - 44, wobei das Adjuvans ein Protein bindet, das mit dem $\alpha$2-Macroglobulin-Rezeptor und dem Rezeptor für Lipoprotein mit niedriger Dichte in Beziehung steht.

**48.** Verwendung nach einem der Ansprüche 1 - 44, wobei die Immunantwort keine allergische Reaktion, Dermatitis oder eine atopische Reaktion darstellt.

**49.** Pflaster für die Verwendung zur transkutanen Immunisierung, wobei das Pflaster ein Antigen und ein Adjuvans umfasst, und wobei die Anwendung des Pflasters auf der Haut des Probanden eine Immunantwort auslöst, die für das Antigen spezifisch ist.

**50.** Pflaster für die Verwendung nach einem der Ansprüche 1 - 48.

**51.** Pflaster nach Anspruch 50, wobei die Anwendung dieses Pflasters die Formulierung hydratisiert.

**52.** Verwendung nach einem der Ansprüche 1 - 20, 28, 29, 35 und 40 - 44, wobei das Antigen durch rekombinante Mittel exprimiert wird.

**53.** Verwendung nach Anspruch 52, wobei das auf rekombinante Weise exprimierte Antigen ein Fusionsprotein ist, das einen Affinitäts-Anhang (affinity tag) oder einen Epitop-Anhang (epitope tag) umfasst.

**54.** Verwendung nach Anspruch 30, wobei der Virus ein Adeno-Virus ist.

**55.** Verwendung nach Anspruch 30, wobei der Virus aus der Gruppe ausgewählt wird, die aus dem Hepatitis-Virus mit dem Serotyp A, B, C, D, E oder aus Kombinationen derselben besteht.

**56.** Verwendung nach Anspruch 30, wobei der Virus ein humanes Immunschwäche-Virus (HI-Virus) ist.

**57.** Verwendung nach Anspruch 30, wobei der Virus ein Papilloma-Virus ist.

**Revendications**

**1.** Utilisation d'un antigène et d'un adjuvant dans la fabrication d'une formulation pour l'immunisation transcutanée d'un sujet, dans laquelle ladite formulation induit une réponse immunitaire spécifique de l'antigène après application de la formulation à la peau du sujet, et en outre dans laquelle la formulation ne contient pas d'amplificateur de pénétration.

**2.** Utilisation selon la revendication 1, dans laquelle l'adjuvant est choisi dans le groupe constitué par un ADN bactérien, les cytokines, les chimiokines et les lipopolysaccharides, une molécule contenant des motifs CpG non méthylés, une protéine de choc thermique, un dérivé d'une protéine de choc thermique et leurs combinaisons.

**3.** Utilisation selon la revendication 1, dans laquelle la formulation comprend en outre un pansement pour former un timbre pour immunisation transcutanée.

**4.** Utilisation selon la revendication 3, dans laquelle le pansement est un pansement occlusif.

**5.** Utilisation selon la revendication 1, dans laquelle une quantité d'antigène efficace pour induire la réponse immunitaire n'est pas encapsulée dans un liposome.

**6.** Utilisation selon la revendication 1, dans laquelle l'adjuvant amplifie la présentation de l'antigène à un lymphocyte.

**7.** Utilisation selon la revendication 1, dans laquelle l'adjuvant active une cellule présentant l'antigène.

**8.** Utilisation selon la revendication 7, dans laquelle la cellule présentant l'antigène est une cellule de Langerhans ou une cellule dendritique dermique.

**9.** Utilisation selon la revendication 1, dans laquelle l'adjuvant augmente l'expression du complexe majeur d'histocompatibilité de classe II sur une cellule présentant l'antigène.

**10.** Utilisation selon la revendication 9, dans laquelle la cellule présentant l'antigène est une cellule de Langerhans ou une cellule dendritique dermique.

**11.** Utilisation selon la revendication 1, dans laquelle l'adjuvant provoque la migration d'une cellule présentant l'antigène sous-jacente au site d'application vers un ganglion lymphatique de drainage.

**12.** Utilisation selon la revendication 11, dans laquelle la cellule présentant l'antigène est une cellule de Langerhans ou une cellule dendritique dermique.

**13.** Utilisation selon la revendication 1, dans laquelle l'adjuvant envoie un signal à une cellule de Langerhans pour qu'elle mûrisse en une cellule dendritique.

**14.** Utilisation selon la revendication 1, dans laquelle la formulation est constituée essentiellement d'antigène et d'adjuvant.

**15.** Utilisation selon la revendication 1, dans laquelle une molécule unique sert à la fois d'antigène et d'adjuvant.

**16.** Utilisation selon la revendication 15, dans laquelle la formulation est constituée essentiellement d'un seul composant qui est à la fois un antigène et un adjuvant.

**17.** Utilisation selon la revendication 1, dans laquelle l'antigène n'a pas de propriétés d'adjuvant.

**18.** Utilisation selon la revendication 1, dans laquelle la formulation est une solution aqueuse.

**19.** Utilisation selon la revendication 1, dans laquelle la formulation ne contient pas de solvant organique.

**20.** Utilisation selon la revendication 1, dans laquelle la formulation est mise sous la forme d'une crème, d'une émulsion, d'un gel, d'une lotion, d'une pommade, d'une pâte ou d'une suspension.

**21.** Utilisation selon la revendication 1, dans laquelle l'antigène est au moins partiellement purifié et dérive d'un pathogène choisi dans le groupe constitué par les bactéries, les virus, les champignons et les parasites.

**22.** Utilisation selon la revendication 1, dans laquelle l'antigène est un antigène de tumeur au moins partiellement purifié.

**23.** Utilisation selon la revendication 1, dans laquelle l'antigène est un auto-antigène au moins partiellement purifié.

**24.** Utilisation selon la revendication 1, dans laquelle l'antigène est un allergène au moins partiellement purifié.

**25.** Utilisation selon la revendication 1, dans laquelle l'antigène est au moins partiellement purifié et a un poids moléculaire supérieur à 500 daltons.

**26.** Utilisation selon la revendication 1, dans laquelle l'antigène est au moins partiellement purifié et a un poids moléculaire supérieur 800 daltons.

**27.** Utilisation selon la revendication 1, dans laquelle l'antigène est au moins partiellement purifié et a un poids moléculaire supérieur 1000 daltons.

**28.** Utilisation selon l'une quelconque des revendications 25 à 27, dans laquelle l'antigène est protéique.

**29.** Utilisation selon l'une quelconque des revendications 25 à 27, dans laquelle l'antigène est un conjugué avec un polysaccharide.

**30.** Utilisation selon la revendication 1, dans laquelle l'antigène est fourni sous la forme d'une cellule entière, d'une bactérie, d'un virion, d'un virus vivant, d'un virus atténué, d'un virus inactivé ou d'un virosome.

**31.** Utilisation selon la revendication 30, dans laquelle le virus est le virus de la rage.

**32.** Utilisation selon la revendication 30, dans laquelle le virus est le virus de la grippe.

**33.** Utilisation selon la revendication 30, dans laquelle la bactérie est l'anthrax.

**34.** Utilisation selon la revendication 30, dans laquelle la bactérie est *E. coli* entérotoxinogène.

**35.** Utilisation selon la revendication 1, dans laquelle la formulation contient au moins deux antigènes différents.

**36.** Utilisation selon la revendication 1, dans laquelle l'antigène est fourni sous la forme d'un acide nucléique comprenant une séquence codant l'antigène, l'adjuvant est fourni sous la forme d'un acide nucléique comprenant une séquence codant l'adjuvant, ou bien l'antigène et l'adjuvant sont tous les deux fournis sous la forme d'un acide nucléique comprenant des séquences codant chacun d'eux.

**37.** Utilisation selon la revendication 36, dans laquelle l'acide nucléique est non-intégratif et non-réplicatif.

**38.** Utilisation selon la revendication 36, dans laquelle l'acide nucléique comprend en outre une région régulatrice liée de façon opérationnelle à la séquence codant l'antigène et/ou l'adjuvant.

**39.** Utilisation selon la revendication 36, dans laquelle la formulation ne contient pas une particule virale, un liposome ou un lipide chargé.

**40.** Utilisation selon la revendication 1, comprenant en outre l'utilisation d'un agent hydratant.

**41.** Utilisation selon la revendication 1, comprenant en outre l'utilisation d'un humectant.

**42.** Utilisation selon la revendication 41, dans laquelle l'humectant est le glycérol.

**43.** Utilisation selon la revendication 1, dans laquelle la formulation comprend une quantité d'antigène efficace pour induire la réponse immunitaire, lequel antigène est en solution et/ou est associé avec un liposome, mais n'est pas encapsulé dans celui-ci.

**44.** Utilisation selon la revendication 1, dans laquelle l'antigène est un glucide, un glycolipide, une glycoprotéine, un lipide, une lipoprotéine, un phospholipide, un polypeptide, une protéine, ou leurs conjugués.

**45.** Utilisation selon l'une quelconque des revendications 1 à 44, dans laquelle l'adjuvant fixe un récepteur sur les cellules présentant l'antigène.

**46.** Utilisation selon l'une quelconque des revendications 1 à 44, dans laquelle l'adjuvant fixe un ganglioside $GM_1$.

**47.** Utilisation selon l'une quelconque des revendications 1 à 44, dans laquelle l'adjuvant fixe une protéine apparentée au récepteur de lipoprotéine basse densité/macroglobuline $\alpha_2$.

**48.** Utilisation selon l'une quelconque des revendications 1 à 44, dans laquelle la réponse immunitaire n'est pas une réaction allergique, une dermatite ou une réaction atopique.

**49.** Patch à utiliser en immunisation transcutanée, le patch comprenant un antigène et un adjuvant, dans lequel l'application du patch sur la peau d'un sujet induit une réponse immunitaire spécifique de l'antigène.

**50.** Patch pour une utilisation selon l'une quelconque des revendications 1 à 48.

**51.** Patch selon la revendication 50, dans lequel l'application dudit patch hydrate la formulation.

**52.** Utilisation selon l'une quelconque des revendications 1-20, 28, 29, 35 et 40-44, dans laquelle l'antigène est exprimé par recombinaison.

**53.** Utilisation selon la revendication 52, dans laquelle l'antigène exprimé par recombinaison est une protéine de fusion comprenant une étiquette d'affinité ou une étiquette épitopique.

**54.** Utilisation selon la revendication 30, dans laquelle le virus est un adénovirus.

**55.** Utilisation selon la revendication 30, dans laquelle le virus est choisi dans le groupe constitué par les sérotypes A, B, C, D, E de l'hépatite, ou leurs combinaisons.

**56.** Utilisation selon la revendication 30, dans laquelle le virus est le virus de l'immunodéficience humaine.

**57.** Utilisation selon la revendication 30, dans laquelle le virus est un virus de papillome.

# Figure 1